(19) Europäisches Patentamt
European Patent Office
Office européen des brevets

(11) EP 4 227 294 A1

(12) EUROPEAN PATENT APPLICATION
published in accordance with Art. 153(4) EPC

(43) Date of publication:
**16.08.2023 Bulletin 2023/33**

(21) Application number: **21877623.5**

(22) Date of filing: **05.10.2021**

(51) International Patent Classification (IPC):
***C07D 207/452*** *(2006.01)* ***C08F 38/00*** *(2006.01)*
***C08G 65/333*** *(2006.01)* ***C08G 65/48*** *(2006.01)*
***C09D 201/00*** *(2006.01)* ***C09J 201/00*** *(2006.01)*
***B32B 7/027*** *(2019.01)*

(52) Cooperative Patent Classification (CPC):
**B32B 7/027; C07D 207/452; C08F 38/00; C08G 65/333; C08G 65/48; C09D 201/00; C09J 201/00**

(86) International application number:
**PCT/JP2021/036833**

(87) International publication number:
**WO 2022/075324 (14.04.2022 Gazette 2022/15)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **09.10.2020 JP 2020170858**

(71) Applicant: **Daicel Corporation**
**Osaka 530-0011 (JP)**

(72) Inventors:
- **OKANO, Yoshimichi**
  **Tokyo 108-8230 (JP)**
- **NAKATANI, Kouji**
  **Tokyo 108-8230 (JP)**

(74) Representative: **Grünecker Patent- und Rechtsanwälte**
**PartG mbB**
**Leopoldstraße 4**
**80802 München (DE)**

# (54) CURABLE COMPOUND PRODUCT

(57) Provided is a curable compound product having excellent storage stability and moldability and by which a cured product having ultra-high heat resistance can be formed. The curable compound product according to the present disclosure has the following characteristics (a) to (g): (a) A number average molecular weight (calibrated with polystyrene standard) is from 1000 to 15000. (b) A proportion of a structure derived from an aromatic ring in a total amount of the curable compound product is 50 wt.% or greater. (c) Solvent solubility at 23°C is 1 g/100 g or greater. (d) The glass transition temperature is from 80 to 230°C. (e) A viscosity ($\eta_0$) of a 20 wt.% NMP solution obtained by subjecting the curable compound product to a reduced-pressure drying process and then dissolving the reduced-pressure-dried curable compound product in NMP, and a viscosity ($\eta_{10}$) of the 20 wt.% NMP solution after being left to stand for 10 days in a desiccator maintained at 23°C satisfy the Equation (E): $\eta_{10}/\eta_0 < 2$ (E). (f) A molecular skeleton has a sulfonyl group. (g) An exothermic onset temperature is 220°C or higher.

EP 4 227 294 A1

## Description

Technical Field

The present disclosure relates to a curable compound product, and a structure containing a cured product or semi-cured product of the curable compound product. The present disclosure claims rights of priority from the Japanese patent application No. 2020-170858 filed in Japan on October 9, 2020, the contents of which are incorporated herein by reference.

Background Art

Engineering plastics are plastics having, for example, enhanced heat resistance and mechanical properties and are very useful as materials essential for miniaturization, weight reduction, performance enhancement and reliability enhancement of various types of parts. However, engineering plastics need to be heated at high temperatures during melt molding. Moreover, because of low solvent solubility of engineering plastics, it is difficult to subject them to solution casting. Therefore, the inability to easily mold engineering plastics has been an issue.

For example, polyimides described in Patent Document 1 have excellent heat resistance and strength characteristics but are not easily dissolved or melted, and thus it has been difficult to use such polyimides as molding materials in melt-molding, solution casting, or the like, or to use these polyimides as matrix resins for composite materials.

Patent Document 2 describes a curable compound represented by the following formula. Patent Document 2 also indicates that when the curable compound is thermally cured, a cured product excelling in flame retardancy and heat resistance can be obtained.

[Chem. 5]

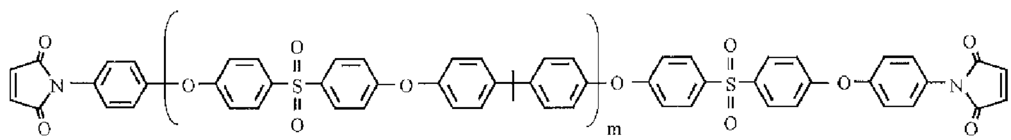

(1-1)

Patent Document 2 discloses that the curable compound is produced by a method in which N-aryl maleic acid is subjected to dehydrative imidization using sodium acetate in the presence of acetic anhydride (see the following formula).

[Chem. 6]

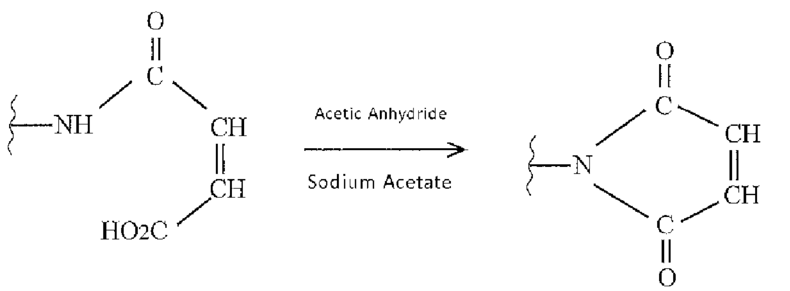


Patent Document 3 discloses that when N-aryl maleic acid is subjected to dehydrative imidization using a base catalyst in the presence of acetic anhydride, a large amount of side reaction products represented by the following Formulas (I-a) and (I-b) are produced together with a bismaleimide represented by the following Formula (I), but when the side reaction products are mixed with the bismaleimide, the curing temperature is lowered, that is, the exothermic onset temperature is lowered.

[Chem. 7]

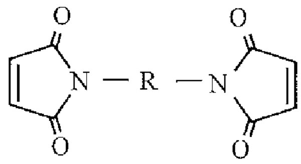

(I)

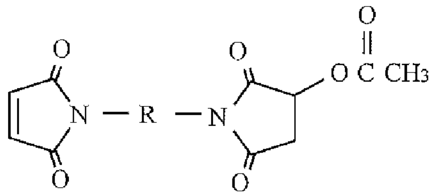

(I-a)

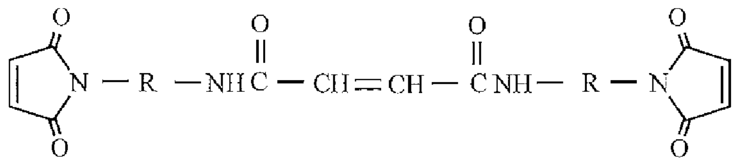

(I-b)

Patent Document 4 discloses that an N-aryl maleic acid is imidized by azeotropic dehydration in the presence of an acid catalyst to produce a curable compound represented by the following formula. Patent Document 4 further indicates that the curable compound represented by the following formula has a low melting temperature and good solvent solubility.

[Chem. 8]

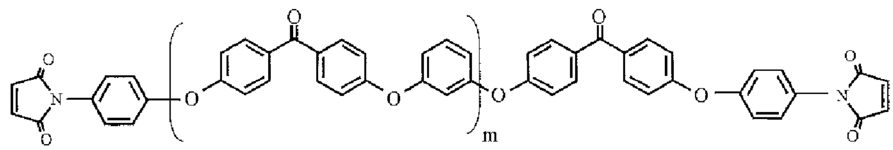

Citation List

Patent Documents

Patent Document 1: JP 2000-219741 A
Patent Document 2: JP 48-36163 A
Patent Document 3: JP 62-151414 A
Patent Document 4: WO 2019/244694

Summary of Invention

Technical Problem

However, with the curable compound product obtained by the method described in Patent Document 2 in which N-aryl maleic acid is subjected to dehydrative imidization using sodium acetate in the presence of acetic anhydride, the exothermic onset temperature is reduced by the mixing in of side reaction products as described in Patent Document 3, and therefore if the curable compound product is subjected to melt molding, the curing reaction proceeds in a nozzle for injecting the curable compound product into a mold or the like, and the nozzle may become clogged.

Furthermore, while the side reaction products are not mixed into the curable compound product obtained by the method described in Patent Document 4, the storage stability of a solution obtained by dissolving the curable

compound product in a solvent is poor, and when the curable compound product is subjected to film molding using a solution casting method, the prepared solution thickens during storage, making it difficult to mold the product into a film shape.

Thus, an object of the present disclosure is to provide a curable compound product excelling in moldability and storage stability as a solution and by which a cured product having ultra-high heat resistance can be formed.

Another object of the present disclosure is to provide a molded product containing a cured product or semi-cured product having ultra-high heat resistance.

Yet another object of the present disclosure is to provide a laminate having a configuration in which a substrate and a cured product or semi-cured product having ultra-high heat resistance are laminated.

Yet another object of the present disclosure is to provide a composite material containing fibers and a cured product or semi-cured product having ultra-high heat resistance.

Yet another object of the present disclosure is to provide an adhesive, a paint, or a sealing agent having excellent storage stability in solution and exhibiting ultra-high heat resistance by being subjected to a heating treatment.

The term "product", as used herein, means a form that is industrially produced and distributed to the market and does not mean a chemical entity itself. Accordingly, the "curable compound product" according to an embodiment of the present disclosure is a collective body in which a plurality of curable compounds are collected, and, in this sense, the "curable compound product" is a composition.

Solution to Problem

As a result of diligent research, the present inventors found that the issues described above can be solved by a curable compound product having the following characteristics (a) to (g). The present disclosure was completed based on these findings.

Specifically, an embodiment of the present disclosure provides a curable compound product including the following characteristics (a) to (g):

(a) a number average molecular weight (calibrated with polystyrene standard) is from 1000 to 15000;
(b) a proportion of a structure derived from an aromatic ring in a total amount of the curable compound is 50 wt.% or greater;
(c) solvent solubility at 23°C is 1 g/100 g or greater;
(d) a glass transition temperature is from 80 to 230°C; and
(e) a viscosity ($\eta_0$) of a 20 wt.% NMP solution obtained by subjecting the curable compound product to a reduced-pressure drying process and then dissolving the reduced-pressure-dried curable compound product in NMP, and a viscosity ($\eta_{10}$) of the 20 wt.% NMP solution after being left to stand for 10 days in a desiccator maintained at 23°C satisfy Equation (E) below:

$$\eta_{10}/\eta_0 < 2 \quad (E)$$

(f) a molecular skeleton has a sulfonyl group; and
(g) an exothermic starting temperature is 220°C or higher.

An embodiment of the present disclosure also provides a curable compound product containing a compound represented by Formula (1) below:

[Chem. 1]

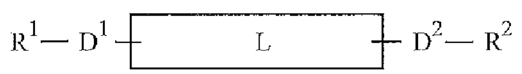

(1)

where in Formula (1), $R^1$ and $R^2$ are identical or different and each represent a group represented by Formula (r-1) below or a group represented by Formula (r-2) below:

[Chem. 2]

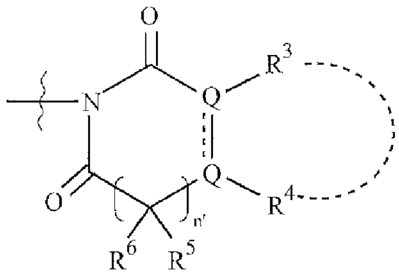

(r-1)

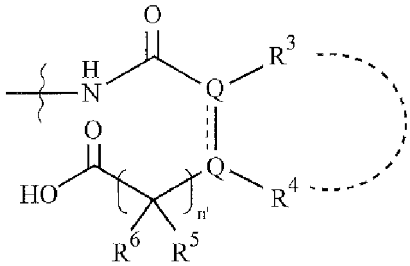

(r-2)

where in Formula (r-1) and Formula (r-2), Q represents C or CH, and in each formula, two Q's bond to each other via a single bond or a double bond; $R^3$ to $R^6$ are identical or different, and each represent a hydrogen atom or a hydrocarbon group; $R^3$ and $R^4$ may bond to each other to form a ring; n' represents an integer of 0 or greater; and a bond indicated by a wavy line in each formula is bonded to $D^1$ or $D^2$, and in Formula (1), $D^1$ and $D^2$ are identical or different, and each represent a single bond or a linking group; L represents a divalent group having a repeating unit containing a structure represented by Formula (I) below and a structure represented by Formula (II) below:

[Chem. 3]

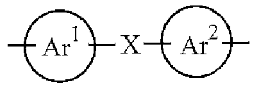

(I)

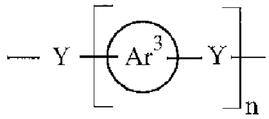

(II)

where in Formula (I) and Formula (II), $Ar^1$ to $Ar^3$ are identical or different, and each represent a group in which two hydrogen atoms are removed from a structure of an aromatic ring or a group in which two hydrogen atoms are removed from a structure containing two or more aromatic rings, the aromatic rings being bonded through a single bond or a linking group; X represents -CO-, -S-, or $-SO_2-$; Y is identical or different, and each represents -S-, $-SO_2-$, -O-, -CO-, -COO-, or -CONH-; and n represents an integer of 0 or greater; with the proviso that, of all X's and Y's included in L, at least one is $-SO_2-$, in which a proportion of the group represented by Formula (r-1) above to a sum of the group represented by Formula (r-1) above and the group represented by Formula (r-2) above is 97% or greater.

An embodiment of the present disclosure also provides the abovementioned curable compound product, in which $D^1$ and $D^2$ in Formula (1) are identical or different, and each represent a group selected from groups having structures represented by Formulas (d-1) to (d-4) below:

[Chem. 4]

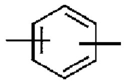

(d-1)

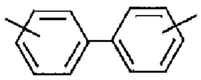

(d-2)

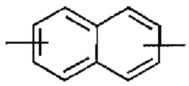

(d-3)

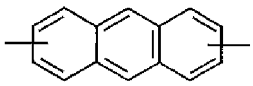

(d-4)

An embodiment of the present disclosure also provides the abovementioned curable compound product, in which $Ar^1$ to $Ar^3$ in Formula (I) and Formula (II) are identical or different, and each represent a group in which two hydrogen atoms are removed from a structure of an aromatic ring having from 6 to 14 carbons, or a group in which two hydrogen atoms are removed from a structure containing two or more aromatic rings each having from 6 to 14 carbons, the aromatic rings being bonded through a single bond, a linear or branched-chain alkylene group having from 1 to 5 carbons, or a group in which one or more hydrogen atoms of a linear or branched-chain alkylene group having from 1 to 5 carbons are substituted with a halogen atom.

An embodiment of the present disclosure also provides a molded product including a cured product or semi-cured product of the curable compound product.

An embodiment of the present disclosure also provides a laminate having a configuration in which a cured product or semi-cured product of the curable compound product and a substrate are laminated.

An embodiment of the present disclosure also provides a method of producing a laminate, the method including placing the curable compound product on a substrate and subjecting to a heating treatment to form a laminate having a configuration in which a cured product or semi-cured product of the curable compound product and the substrate are laminated.

An embodiment of the present disclosure also provides the abovementioned method of producing a laminate, the method comprising applying a molten material of the curable compound product onto a support made of plastic, solidifying the applied material to obtain a thin film containing the curable compound product, detaching the formed thin film from the support, laminating the formed thin film on a substrate, and subjecting to a heating treatment.

An embodiment of the present disclosure also provides a composite material including a cured product or semi-cured product of the curable compound product and fibers.

An embodiment of the present disclosure also provides an adhesive including the curable compound product.

An embodiment of the present disclosure also provides a paint including the curable compound product.

An embodiment of the present disclosure also provides a sealing agent including the curable compound product.

Advantageous Effects of Invention

The curable compound product according to an embodiment of the present disclosure has excellent solvent solubility. Furthermore, when the curable compound product is dissolved in a solvent, a solution having a viscosity suitable for film forming by a solution casting method can be obtained. Furthermore, the solution can be stored for a long period of time while suppressing thickening. That is, a solution obtained by dissolving the curable compound product in a solvent has high storage stability.

Moreover, the melt viscosity of the curable compound product is low, and the exothermic onset temperature is high. Therefore, when the curable compound product is subjected to melting and molding, the degree of freedom in setting conditions relating to molding is high, curing during injection into a mold or the like can be prevented, and moldability is excellent.

Furthermore, when the curable compound product is subjected to a heating treatment, a cured product having ultra-high heat resistance can be obtained. In addition, the cured product that is obtained also exhibits excellent toughness.

Therefore, the curable compound product can be suitably used in an adhesive, a sealing agent, a paint, or the like in a field where ultra-high heat resistance is required (e.g., electronic information devices, home appliances, automobiles, precision machines, aircraft, devices for the space industry, etc.).

## Brief Description of Drawings

FIG. 1 illustrates $^1$H-NMR measurement results of a compound product (1-5) obtained in an example and of a compound product (1-6) obtained in a comparative example.

FIG. 2 is a graph illustrating TGA measurement results of compound products (1-4) and (1-5) obtained in the examples.

## Description of Embodiments

### Curable Compound Product

A curable compound product according to an embodiment of the present disclosure includes the following characteristics (a) to (g):

(a) a number average molecular weight (calibrated with polystyrene standard) is from 1000 to 15000;
(b) a proportion of a structure derived from an aromatic ring in a total amount of the curable compound product is 50 wt.% or greater;
(c) solvent solubility at 23°C is 1 g/100 g or greater;
(d) a glass transition temperature is from 80 to 230°C;
(e) a viscosity ($\eta_0$) of a 20 wt.% NMP solution obtained by subjecting the curable compound product to a reduced-pressure drying process and then dissolving the reduced-pressure-dried curable compound product in NMP, and a viscosity ($\eta_{10}$) of the 20 wt.% NMP solution after being left to stand for 10 days in a desiccator maintained at 23°C satisfy Equation (E) below:

$$\eta_{10}/\eta_0 < 2 \quad (E)$$

(f) the molecular skeleton has a sulfonyl group ($-SO_2-$); and
(g) the exothermic starting temperature is 220°C or higher.

The curable compound product may further have the following characteristics (h) and (i):

(h) a 5% weight loss temperature (Tas) measured at a temperature increase rate of 20°C/min in a nitrogen atmosphere is 400°C or higher; and
(i) the nitrogen atom content is from 2.8 to 0.1 wt.%.

A cured product of the curable compound product may have the following characteristics (j) to (m):

(j) a 5% weight loss temperature (Tas) measured at a temperature increase rate of 20°C/min (in nitrogen) is 400°C or higher;
(k) the elastic modulus (measured by a method according to JIS K7161) is 1000 MPa or greater (preferably 1150 MPa or greater, and particularly preferably 1200 MPa or greater);
(l) the yield point stress (measured by a method according to JIS K7161) is 70 MPa or greater (preferably 80 MPa or greater); and
(m) the elongation at break (measured by a method according to JIS K7161) is 10% or greater.

The number average molecular weight (Mn) of the curable compound product is from 1000 to 15000. The lower limit of the number average molecular weight (Mn) is preferably 1500, further preferably 2000, particularly preferably 2200, most preferably 2600, and especially preferably 2700. The upper limit of the number average molecular weight (Mn) is preferably 12000, more preferably 10000, further preferably 7000, still further preferably 5000, particularly preferably 4000, and especially preferably 3600.

The weight average molecular weight (Mw) of the curable compound product is, for example, from 1000 to 10000. The lower limit of the weight average molecular weight (Mw) is preferably 2000, further preferably 2500, particularly preferably 3000, and most preferably 4000. The upper limit of the weight average molecular weight (Mw) is preferably

8000, more preferably 7500, particularly preferably 7000, and most preferably 6500.

Because the curable compound product has the molecular weight described above, solubility of the curable compound product in a solvent is high, and the melt viscosity is low. Therefore, the curable compound product is easy to mold. Furthermore, the obtained cured product (or molded body after curing) exhibits a high level of toughness. When the molecular weight is less than the range described above, toughness of the resulting cured product tends to decrease. On the other hand, when the molecular weight is greater than the range described above, solvent solubility tends to decrease, melt viscosity tends to increase excessively, and workability tends to decrease. Note that Mn is determined by gel permeation chromatography (GPC) measurement (solvent: chloroform; calibrated with polystyrene standard).

A proportion of the structure derived from an aromatic ring in the total amount of the curable compound product is 50 wt.% or greater, preferably 60 wt.% or greater, and particularly preferably 65 wt.% or greater. Note that the upper limit of the abovementioned proportion is, for example, 95 wt.%. Therefore, the curable compound product has a high solvent solubility and a low melt viscosity, and a cured product thereof has a high thermal stability. A proportion of the structure derived from an aromatic ring less than the range described above tends to reduce thermal stability of the cured product. On the other hand, a proportion of the structure derived from an aromatic ring greater than the range described above tends to reduce solvent solubility, increase melt viscosity, and reduce workability.

The nitrogen atom content of the curable compound product is, for example, from 0.1 to 2.8 wt.%, preferably from 0.2 to 2.5 wt.%, more preferably from 0.25 to 2.0 wt.%, and particularly preferably from 0.3 to 1.8 wt.%. The nitrogen atom content can be determined by CHN elemental analysis. The curable compound product having a nitrogen atom content in the range described above achieves excellent solvent solubility and can form a cured product having excellent toughness and heat resistance. Meanwhile, when the nitrogen atom content is less than the range described above, formation of a cured product having excellent toughness and heat resistance tends to be difficult. Furthermore, when the nitrogen atom content is greater than the range described above, solvent solubility tends to decrease.

The curable compound product has a sulfonyl group in its molecular skeleton. In relation to the total amount of the curable compound product, the sulfonyl group content is, for example, from 12.4 to 19.4 wt.%, preferably from 13.0 to 18.5 wt.%, and particularly preferably from 13.9 to 17.9 wt.%. The sulfonyl group content can be determined by CHN elemental analysis. A curable compound product in which the sulfonyl group content is within the abovementioned range has excellent solvent solubility. On the other hand, when the sulfonyl group content is less than the range described above, the solvent solubility tends to decrease. In addition, when the sulfonyl group content exceeds the range described above, formation of a cured product having excellent toughness and heat resistance tends to be difficult.

The curable compound product exhibits good solvent solubility. Examples of the solvent include chain ketones, such as methyl ethyl ketone (MEK) and methyl isobutyl ketone; cyclic ketones, such as cyclohexanone (ANON) and cycloheptanone; amides, such as formamide, acetamide, N-methyl-2-pyrrolidone (NMP), N,N-dimethylformamide, and dimethylacetamide (DMAc); halogenated hydrocarbons, such as methylene chloride, chloroform, 1,2-dichloroethane, chlorobenzene, bromobenzene, dichlorobenzene, benzotrifluoride, and hexafluoro-2-propanol; sulfoxides, such as dimethylsulfoxide (DMSO), diethyl sulfoxide, and benzyl phenyl sulfoxide; tetrahydrofuran (THF); aromatic hydrocarbons, such as benzene, toluene, and xylene; and liquid mixtures of two or more types of these. The curable compound product exhibits excellent solubility, in particular, in at least one type of solvent selected from ether, chain ketones, cyclic ketones, amides, halogenated hydrocarbons, and sulfoxides.

The solubility of the curable compound product in a solvent is 1 g or greater, preferably 5 g or greater, and particularly preferably 10 g or greater, per 100 g of the solvent at 23°C.

Therefore, when the solution obtained by dissolving the curable compound product in a solvent is formed into a film shape by a casting method and cured, a film-shaped cured product can be formed.

Furthermore, a 20 wt.% NMP solution obtained by subjecting the curable compound product to a reduced-pressure drying process and then dissolving it in NMP has a low viscosity and is suitable for solution casting. The viscosity ($\eta_0$) of the 20 wt.% NMP solution is from 5 to 1000 mPa·s, for example. The lower limit of the viscosity ($\eta_0$) is preferably 10 mPa·s, and particularly preferably 15 mPa·s. The upper limit of the viscosity ($\eta_0$) is preferably 700 mPa·s, more preferably 500 mPa s, further preferably 300 mPa s, further preferably 200 mPa s, further preferably 150 mPa s, further preferably 100 mPa·s, particularly preferably 70 mPa·s, most preferably 50 mPa·s, and especially preferably 40 mPa·s. If the viscosity ($\eta_0$) is too low, it is difficult to mold a thick film, and if the viscosity is too high, molding failure such as thickness unevenness tends to be easily caused.

The curable compound product has excellent storage stability, and the viscosity ($\eta_{10}$) of a 20 wt.% NMP solution after being left to stand for 10 days in a desiccator maintained at 23°C is from 10 to 2000 mPa s, for example, the 20 wt.% NMP solution being obtained by subjecting the curable compound product to a reduced-pressure drying process and then dissolving the reduced-pressure-dried curable compound product in NMP. The lower limit of the viscosity ($\eta_{10}$) is preferably 20 mPa s, particularly preferably 30 mPa s, and most preferably 400 mPa s. The upper limit of the viscosity ($\eta_{10}$) is preferably 1400 mPa s, more preferably 1000 mPa·s, further preferably 600 mPa·s, particularly preferably 400 mPa·s, most preferably 300 mPa·s, and especially preferably 200 mPa·s.

Furthermore, the viscosity ($\eta_0$) and the viscosity ($\eta_{10}$) preferably satisfy the Equation (E) below:

$$\eta_{10}/\eta_0 < 2 \text{ (E)}$$

The viscosity ($\eta_0$) and the viscosity ($\eta_{10}$) more preferably satisfy the Equation (E-1) below, and particularly preferably satisfy the Equation (E-2) below:

$$\eta_{10}/\eta_0 < 1.75 \text{ (E-1)}$$

$$\eta_{10}/\eta_0 < 1.5 \text{ (E-2)}$$

The viscosity ($\eta_0$) and the viscosity ($\eta_{10}$) preferably satisfy the Equation (E-3) below:

$$1 \leq \eta_{10}/\eta_0 < 2 \text{ (E-3)}$$

Note that the viscosity of the NMP solution is the viscosity at 23°C and normal pressure, and can be measured using an E-type viscometer. Furthermore, the reduced-pressure drying process of the curable compound product is performed until the amount of residual moisture in the curable compound product is 0.05% or less.

The 5% weight loss temperature (Tas) of the curable compound product measured at a temperature increase rate of 20°C/min (in nitrogen) is 400°C or higher, preferably 450°C or higher, and most preferably 500°C or higher. The upper limit of the 5% weight loss temperature (Tas) is, for example, 600°C, preferably 550°C, and particularly preferably 530°C.

The 10% weight loss temperature ($T_{d10}$) of the curable compound product measured at a temperature increase rate of 20°C/min (in nitrogen) is, for example, 300°C or higher, preferably 400°C or higher, particularly preferably 480°C or higher, and most preferably 500°C or higher. The upper limit of the 10% weight loss temperature ($T_{d10}$) is, for example, 600°C, and preferably 550°C.

The 5% weight loss temperature and the 10% weight loss temperature are determined by thermogravimetric (TGA) measurements using TG/DTA (differential thermal and thermal gravimetric measuring instrument).

The glass transition temperature (Tg) of the curable compound product is from 100 to 230°C. The upper limit of the glass transition temperature (Tg) is preferably 220°C, particularly preferably 210°C, and most preferably 200°C. The lower limit of the glass transition temperature (Tg) is preferably 110°C, and particularly preferably 125°C. Note that Tg can be determined by differential scanning calorimetry (DSC).

The curable compound product has a low glass transition temperature (Tg), and thus has excellent melt workability. When the glass transition temperature (Tg) is greater than the range described above, heating at a high temperature is required during melting, and thus workability decreases, and for example, in a case where a composite material is produced by impregnating fibers with the curable compound product in a molten state, it may be difficult to impregnate between fine fibers due to progress of a curing reaction of the curable compound product.

The exothermic onset temperature of the curable compound product is 220°C or higher, preferably 230°C or higher, more preferably 240°C or higher, and particularly preferably 250°C or higher. In addition, the upper limit of the exothermic onset temperature is, for example, 300°C. Therefore, the curable compound product exhibits excellent moldability. Note that the exothermic onset temperature can be measured using DSC at a temperature of temperature increase of 20°C/min (in nitrogen).

When the curable compound product is heated at a temperature equal to or higher than the exothermic onset temperature, the product can rapidly cure to form a cured product having a highly crosslinked structure and having ultra-high heat resistance. Furthermore, because the curing reaction does not proceed at a temperature below the exothermic onset temperature, for example, when molding is performed, the injection of the curable compound product into the mold frame is performed at a temperature lower than the exothermic onset temperature, thereby making it possible to inject the curable compound product while suppressing thickening thereof, and to achieve a stable injection operation and molding with high precision.

Note that heating may be performed while the temperature is maintained constant or may be performed by changing the temperature stepwise. The heating temperature can be appropriately adjusted depending on the heating time and, for example, in the case where shortening of the heating time is desired, the heating temperature is preferably set high. Because a high proportion of the curable compound product is a structure derived from an aromatic ring, a cured product can be formed without causing decomposition even when heating is carried out at a high temperature, and a cured product can be efficiently formed with superior operation efficiency by heating at a higher temperature for a shorter period of time. Furthermore, the heating means is not particularly limited, and a known and common means

can be used.

Also, when heating at a temperature equal to or higher than the exothermic onset temperature is difficult, and molding through heating at a temperature equal to or lower than the exothermic onset temperature is required, a polymerization initiator such as a radical polymerization initiator may be added to the curable compound product at an approximate amount of from 0.05 to 10.0 parts by weight per 100 parts by weight of the curable compound product. As a result, the cured product can be formed at a lower temperature than the heat generation temperature.

Curing of the curable compound product can be performed under normal pressure, under reduced pressure, or under pressurization.

When the heating temperature and heating time of the curable compound product are adjusted to stop curing reaction in the middle of the reaction without completing the reaction, a semi-cured product (B-stage) can be formed. The degree of cure of the semi-cured product is, for example, 85% or less (e.g., from 10 to 85%, particularly preferably from 15 to 75%, and even more preferably from 20 to 70%).

Note that the degree of cure of the semi-cured product can be calculated from the following equation by measuring the calorific value of the curable compound product and the calorific value of the semi-cured product thereof by DSC.

$$\text{Degree of cure (\%)} = [1 - (\text{calorific value of semi-cured product/calorific value of curable compound product})] \times 100$$

The semi-cured product of the curable compound product can temporarily exhibit fluidity through heating and can conform to a shape such as a step. Furthermore, by further heating, a cured product having excellent heat resistance can be formed.

A 5% weight loss temperature (Tas) of a cured product of the curable compound product measured at a temperature increase rate of 20°C/min (in nitrogen) is 400°C or higher, preferably 450°C or higher, and most preferably 500°C or higher. The upper limit of the 5% weight loss temperature (Tas) is, for example, 600°C, preferably 550°C, and particularly preferably 530°C.

A 10% weight loss temperature ($T_{d10}$) of the cured product of the curable compound product measured at a rate of temperature increase of 20°C/min (in nitrogen) is, for example, 300°C or higher, preferably 400°C or higher, particularly preferably 480°C or higher, and most preferably 500°C or higher. The upper limit of the 10% weight loss temperature ($T_{d10}$) is, for example, 600°C, and preferably 550°C.

The glass transition temperature (Tg) of the cured product of the curable compound product is, for example, from 120 to 250°C. Furthermore, the upper limit of the glass transition temperature (Tg) is preferably 245°C, and particularly preferably 240°C. The lower limit of the glass transition temperature (Tg) is preferably 130°C, and particularly preferably 140°C. Note that Tg can be measured by a DSC method.

Because the curable compound product has the characteristics described above, for example, the curable compound product can be used as molding materials for composite materials to be used in a severe environmental temperature, such as those for electronic information devices, home appliances, automobiles, and precision machines, and as functional materials, such as insulating materials and heat-resistant adhesives. Besides, the curable compound product can be suitably used for sealing agents, paints, adhesives, inks, sealants, resists, and forming materials [forming materials for, for example, substrates, electrical insulation materials (such as electrical insulation films), laminated plates, composite materials (such as fiber-reinforced plastics and prepregs), optical elements (such as lenses), optical shaping materials, electronic papers, touch panels, solar cell substrates, optical waveguide materials, light guide plates, and holographic memories].

Because the curable compound product has the characteristics described above, the curable compound product can be particularly suitably used as a sealing agent that covers a semiconductor element in a highly heat-resistant and highly voltage-resistant semiconductor device (such as power semiconductor), for which employment of a known resin material has been difficult.

Furthermore, because the curable compound product has the characteristics described above, the curable compound product can be suitably used as an adhesive [e.g., heat-resistant adhesive used for laminating a semiconductor in a highly heat-resistant and highly voltage-resistant semiconductor device (such as power semiconductor)].

Furthermore, because the curable compound product has the characteristics described above, the curable compound product can be suitably used as a paint (solvent coating agent or powder coating agent) [e.g., paint (solvent coating agent or powder coating agent) used for a highly heat-resistant and highly voltage-resistant semiconductor device (such as power semiconductor)].

The curable compound product includes a compound represented by Formula (1) below:

[Chem. 9]

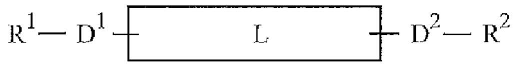

(1)

where in Formula (1), $R^1$ and $R^2$ are identical or different, and each represent a group represented by Formula (r-1) below or a group represented by Formula (r-2) below:

[Chem. 10]

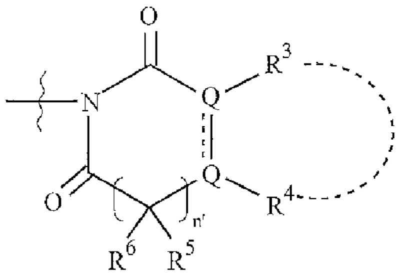

(r-1)

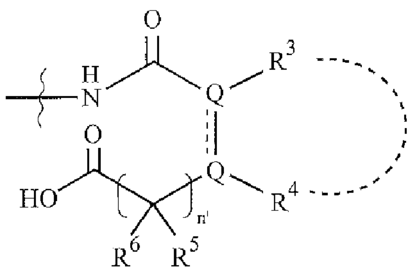

(r-2)

where in Formula (r-2), Q represents C or CH; in each formula, two Q's bond to each other via a single bond or a double bond; $R^3$ to $R^6$ are identical or different, and each represent a hydrogen atom or a hydrocarbon group; $R^3$ and $R^4$ may bond to each other to form a ring; n' represents an integer of 0 or greater; and a bond indicated by a wavy line in the formula is bonded to $D^1$ or $D^2$, and in Formula (1), $D^1$ and $D^2$ are identical or different, and each represent a single bond or a linking group. L represents a divalent group having a repeating unit containing a structure represented by Formula (I) below and a structure represented by Formula (II) below:

[Chem. 11]

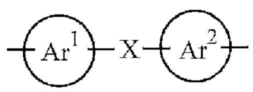

(I)

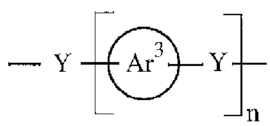

(II)

where in Formula (I) and Formula (II), $Ar^1$ to $Ar^3$ are identical or different, and each represent a group in which two hydrogen atoms are removed from a structure of an aromatic ring or a group in which two hydrogen atoms are removed from a structure containing two or more aromatic rings, the aromatic rings being bonded through a single bond or a linking group; X represents -CO-, -S-, or $-SO_2-$; Y is identical or different, and each represents -S-, $-SO_2-$, -O-, -CO-, -COO-, or -CONH-; and n represents an integer of 0 or greater. However, at least one of all X's and Y's included in L is $-SO_2-$.

In Formulas (r-1) and (r-2) above, Q represents C or CH. Two Q's in each formula bond to each other via a single bond or a double bond.

$R^3$ to $R^6$ are identical or different, and each represent a hydrogen atom, a saturated or unsaturated aliphatic hydrocarbon group (preferably an alkyl group having from 1 to 10 carbons, an alkenyl group having from 2 to 10 carbons,

or an alkynyl group having from 2 to 10 carbons), an aromatic hydrocarbon group (preferably an aryl group having from 6 to 10 carbons, such as a phenyl group or a naphthyl group), or a group in which two or more groups selected from the saturated or unsaturated aliphatic hydrocarbon groups and aromatic hydrocarbon groups described above are bonded.

$R^3$ and $R^4$ may be bonded to each other to form a ring with an adjacent carbon atom. Examples of the ring include alicyclic rings having from 3 to 20 carbons and aromatic rings having from 6 to 14 carbons. Examples of the alicyclic rings having from 3 to 20 carbons include approximately 3 to 20-membered (preferably 3 to 15-membered, particularly preferably 5 to 8-membered) cycloalkane rings, such as a cyclopropane ring, a cyclobutane ring, a cyclopentane ring, and a cyclohexane ring; approximately 3 to 20-membered (preferably 3 to 15-membered, particularly preferably 5 to 8-membered) cycloalkene rings, such as a cyclopentene ring and a cyclohexene ring; and crosslinked cyclic hydrocarbon groups, such as a perhydronaphthalene ring, a norbornane ring, a norbornene ring, an adamantane ring, a tricyclo[5.2.1.$0^{2,6}$]decane ring, and a tetracyclo[4.4.0.$1^{2,5}$.$1^{7,10}$]dodecane ring. The aromatic rings having from 6 to 14 carbons include a benzene ring and a naphthalene ring.

n' is an integer of 0 or greater, and is, for example, an integer from 0 to 3, and preferably 0 or 1.

The group represented by Formula (r-1) above is, in particular, preferably a group selected from groups represented by Formulas (r-1-1) to (r-1-6) below:

[Chem. 12]

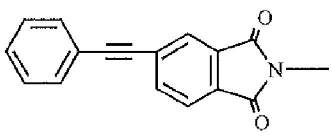

(r-1-1)

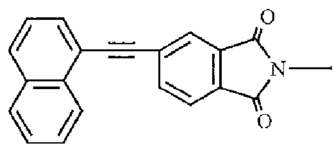

(r-1-2)

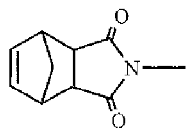

(r-1-3)

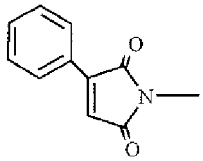

(r-1-4)

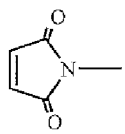

(r-1-5)

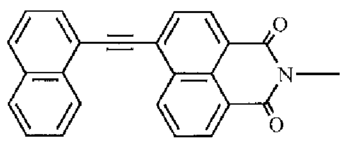

(r-1-6)

A bond from a nitrogen atom in each of the formulas bonds to $D^1$ or $D^2$ in Formula (1).

One or two or more substituents may bond to each of the groups represented by Formulas (r-1-1) to (r-1-6) above. Examples of the substituent include alkyl groups having from 1 to 6 carbons, alkoxy groups having from 1 to 6 carbons, and halogen atoms.

Examples of the alkyl group having from 1 to 6 carbons include linear or branched alkyl groups, such as a methyl group, an ethyl group, a propyl group, an isopropyl group, a butyl group, an isobutyl group, an s-butyl group, a t-butyl group, a pentyl group, and a hexyl group.

Examples of the alkoxy group having from 1 to 6 carbons include linear or branched alkoxy groups, such as a methoxy group, an ethoxy group, a butoxy group, and a t-butyloxy group.

Examples of the group represented by Formula (r-2) above include groups corresponding to the groups of Formulas (r-1-1) to (r-1-6) above, which are obtained by opening of the imide bonds in the groups of Formulas (r-1-1) to (r-1-6) above.

The group represented by Formula (r-1) above and the group represented by Formula (r-2) above are preferably a group represented by Formula (r-1') and a group represented by Formula (r-2'), respectively.

[Chem. 13]

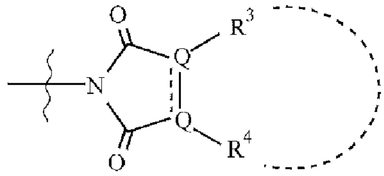

(r-1')

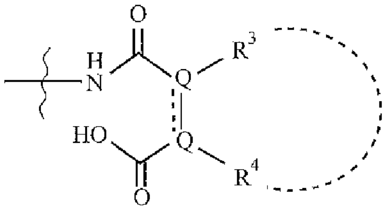

(r-2')

where in Formula (r-1') and Formula (r-2'), Q, $R^3$, and $R^4$ are the same as described above.

The group represented by Formula (r-1) is particularly preferably a group selected from the groups represented by Formulas (r-1-1) to (r-1-5) above (that is, groups including imide bonds), and especially preferably a group represented by Formula (r-1-1) or (r-1-5) above.

The group represented by Formula (r-2) above is preferably a group corresponding to any of the groups represented by Formulas (r-1-1) to (r-1-5) above and obtained by opening of the imide bonds in the groups of Formulas (r-1-1) to (r-1-5) above (that is, group including an amic acid structure), and particularly preferably a group obtained by opening of the imide bonds in the group represented by Formula (r-1-1) or (r-1-5) above.

In Formula (1), $D^1$ and $D^2$ are identical or different, and each represent a single bond or a linking group. Examples of the linking group include divalent hydrocarbon groups, divalent heterocyclic groups, a carbonyl group, an ether bond, an ester bond, a carbonate bond, an amido bond, an imido bond, and groups made by linking a plurality of these.

The divalent hydrocarbon group includes a divalent aliphatic hydrocarbon group, a divalent alicyclic hydrocarbon group, and a divalent aromatic hydrocarbon group.

Examples of the divalent aliphatic hydrocarbon group include linear or branched alkylene groups having from 1 to 18 carbons and linear or branched alkenylene groups having from 2 to 18 carbons. Examples of the linear or branched alkylene group having from 1 to 18 carbons include a methylene group, a methyl methylene group, a dimethyl methylene group, an ethylene group, a propylene group, and a trimethylene group. Examples of the linear or branched alkenylene group having from 2 to 18 carbons include a vinylene group, a 1-methylvinylene group, a propenylene group, a 1-butenylene group, and a 2-butenylene group.

The divalent alicyclic hydrocarbon group include divalent alicyclic hydrocarbon groups having from 3 to 18 carbons, and examples thereof include cycloalkylene groups (including cycloalkylidene groups), such as a 1,2-cyclopentylene group, a 1,3-cyclopentylene group, a cyclopentylidene group, a 1,2-cyclohexylene group, a 1,3-cyclohexylene group, a 1,4-cyclohexylene group, and a cyclohexylidene group.

Examples of the divalent aromatic hydrocarbon group include arylene groups having from 6 to 14 carbons, and examples thereof include a 1,4-phenylene group, a 1,3-phenylene group, a 4,4'-biphenylene group, a 3,3'-biphenylene group, a 2,6-naphthalenediyl group, a 2,7-naphthalenediyl group, a 1,8-naphthalenediyl group, and an anthracenediyl group.

Heterocycles constituting the divalent heterocyclic groups include aromatic heterocycles and nonaromatic heterocycles. Examples of such a heterocycle include 3 to 10-membered rings (preferably 4 to 6-membered rings) having carbon atoms and at least one heteroatom (e.g., oxygen atom, sulfur atom, or nitrogen atom) as atoms constituting the ring, and condensed rings thereof. Specific examples thereof include heterocycles containing an oxygen atom as a heteroatom (e.g., 3-membered rings, such as an oxirane ring; 4-membered rings, such as an oxetane ring; 5-membered rings, such as a furan ring, a tetrahydrofuran ring, an oxazole ring, an isoxazole ring, and a $\gamma$-butyrolactone ring; 6-membered rings, such as a 4-oxo-4H-pyran ring, a tetrahydropyran ring, and a morpholine ring; condensed rings, such as a benzofuran ring, an isobenzofuran ring, a 4-oxo-4H-chromene ring, a chroman ring, and an isochroman ring; crosslinked rings, such as a 3-oxatricyclo[4.3.1.1$^{4,8}$]undecan-2-one ring and a 3-oxatricyclo[4.2.1.0$^{4,8}$]nonan-2-one ring), heterocycles containing a sulfur atom as a heteroatom (e.g., 5-membered rings, such as a thiophene ring, a thiazole ring, an isothiazole ring, and a thiadiazole ring; and 6-membered rings, such as a 4-oxo-4H-thiopyran ring; condensed rings, such as a benzothiophene ring), and heterocycles containing a nitrogen atom as a heteroatom (e.g., 5-membered rings, such as a pyrrole ring, a pyrrolidine ring, a pyrazole ring, an imidazole ring, and a triazole ring; 6-membered rings, such as an isocyanuric ring, a pyridine ring, a pyridazine ring, a pyrimidine ring, a pyrazine ring, a piperidine ring, and a piperazine ring; condensed rings, such as an indole ring, an indoline ring, a quinoline ring, an acridine ring, a naphthyridine ring, a quinazoline ring, and a purine ring). The divalent heterocyclic group is a group obtained by removing two hydrogen atoms from the heterocycle structure described above.

$D^1$ and $D^2$ described above are especially preferably groups including a divalent aromatic hydrocarbon group, from the perspective of forming a cured product having outstanding heat resistance. The divalent aromatic hydrocarbon group is preferably a divalent aromatic hydrocarbon group having from 6 to 14 carbons, more preferably a group selected from groups represented by Formulas (d-1) to (d-4) below, particularly preferably a group represented by Formula (d-1) below (1,2-phenylene group, 1,3-phenylene group, or 1,4-phenylene group), and most preferably 1,4-phenylene group.

[Chem. 14]

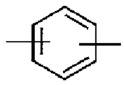
(d-1)

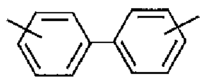
(d-2)

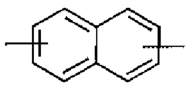
(d-3)

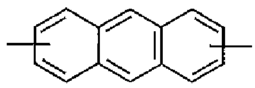
(d-4)

Furthermore, $D^1$ and $D^2$ described above is preferably a group in which, together with the divalent aromatic hydrocarbon group, at least one group selected from the group consisting of a carbonyl group, an ether bond, an ester bond, a carbonate bond, an amido bond, and an imido bond is linked, and especially preferably a group in which an ether bond is linked to the divalent aromatic hydrocarbon group described above.

Thus, the $R^1$-$D^1$- group and the $R^2$-$D^2$- group in Formula (1) are, for example, identical or different, and are each a group selected from groups represented by Formulas (rd-1'-1) to (rd-2'-2) below:

[Chem. 15]

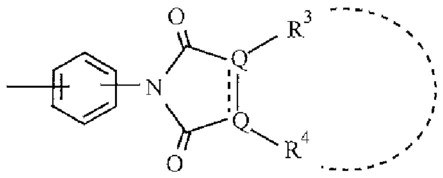

(rd-1'-1)

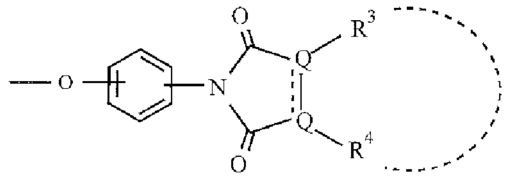

(rd-1'-2)

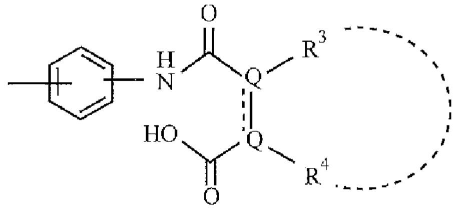

(rd-2'-1)

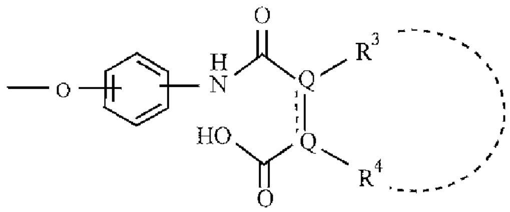

(rd-2'-2)

where in Formulas (rd-1'-1) to (rd-2'-2), Q, $R^3$, and $R^4$ are each the same as described above.

L in Formula (1) represents a divalent group having a repeating unit containing a structure represented by Formula (I) above and a structure represented by Formula (II) above. In Formula (I) and Formula (II), $Ar^1$ to $Ar^3$ are identical or different, and each represent a group in which two hydrogen atoms are removed from a structure of an aromatic ring or a group in which two hydrogen atoms are removed from a structure containing two or more aromatic rings, the aromatic rings being bonded through a single bond or a linking group. X represents -CO-, -S-, or $-SO_2-$;

Y is identical or different, and each represents -S-, $-SO_2-$, -O-, -CO-, -COO-, or -CONH-. However, at least one of all X and Y contained in L is $-SO_2-$.

n in Formula (II) represents an integer of 0 or greater and is, for example, an integer from 0 to 5, preferably an integer from 1 to 5, and particularly preferably an integer from 1 to 3.

Examples of the aromatic ring, which is an aromatic hydrocarbon ring include aromatic rings having from 6 to 14 carbons, such as benzene, naphthalene, anthracene, and phenanthrene. Among these, an aromatic ring having from 6 to 10 carbons, such as benzene or naphthalene, is preferred.

Examples of the linking group include divalent hydrocarbon groups having from 1 to 5 carbons and groups in which one or more hydrogen atoms of a divalent hydrocarbon group having from 1 to 5 carbons is substituted with halogen atom(s).

Examples of the divalent hydrocarbon groups having from 1 to 5 carbons include linear or branched alkylene groups having from 1 to 5 carbons, such as a methylene group, a methylmethylene group, a dimethylmethylene group, a dimethylene group, and a trimethylene group; linear or branched alkenylene groups having from 2 to 5 carbons, such as a vinylene group, 1-methylvinylene group, and a propenylene group; and linear or branched alkynylene groups having from 2 to 5 carbons, such as an ethynylene group, a propynylene group, and 1-methylpropynylene group. Among these, a linear or branched alkylene group having from 1 to 5 carbons is preferred, and a branched alkylene group having from 1 to 5 carbons is particularly preferred.

$Ar^1$ to $Ar^3$ described above are identical or different and, among others, are each preferably a group selected from the groups represented by Formulas (a-1) to (a-5) below. Note that, in the following formulas, positions of bonding are not particularly limited.

[Chem. 16]

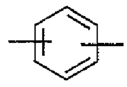
(a-1)

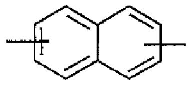
(a-2)

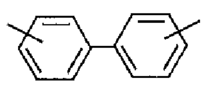
(a-3)

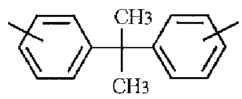
(a-4)

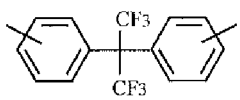
(a-5)

Of these, $Ar^1$ and $Ar^2$ in Formula (I) are each preferably a group represented by Formula (a-1) or (a-2) above.

Furthermore, of these, X is preferably -CO- or $-SO_2-$.

In addition, among these, Y is preferably -S-, $-SO_2-$, -O-, or -CO-.

Therefore, the structure represented by the Formula (I) preferably includes a structure derived from benzophenone and/or a structure derived from diphenyl sulfone, and particularly preferably includes a structure derived from diphenyl sulfone.

The proportion of the structural unit derived from diphenyl sulfone in the total amount of the compound represented by Formula (1) is, for example, 5 wt.% or greater, preferably from 10 to 62 wt.%, and particularly preferably from 15 to 60 wt.%.

In particular, the structure represented by Formula (II) preferably includes a structure derived from at least one compound selected from the group consisting of hydroquinone, resorcinol, 2,6-naphthalenediol, 2,7-naphthalenediol, 4,4'-dihydroxybiphenyl, 4,4'-dihydroxydiphenyl ether, 4,4'-dihydroxybenzophenone, 4,4'-dihydroxydiphenyl sulfide, 4,4'-dihydroxydiphenyl sulfone, and bisphenol A.

The proportion of a structural unit derived from hydroquinone, resorcinol, 2,6-naphthalenediol, 2,7-naphthalenediol, 4,4'-dihydroxybiphenyl, 4,4'-dihydroxydiphenyl ether, 4,4'-dihydroxybenzophenone, 4,4'-dihydroxydiphenyl sulfide, 4,4'-dihydroxydiphenyl sulfone, and bisphenol A in the total amount of the compound represented by Formula (1) is, for example, 5 wt.% or greater, preferably from 10 to 55 wt.%, and particularly preferably from 15 to 53 wt.%.

L in Formula (1) is, in particular, preferably a divalent group represented by Formula (L-1) below from the perspective of being able to obtain a cured product having outstanding heat resistance.

[Chem. 17]

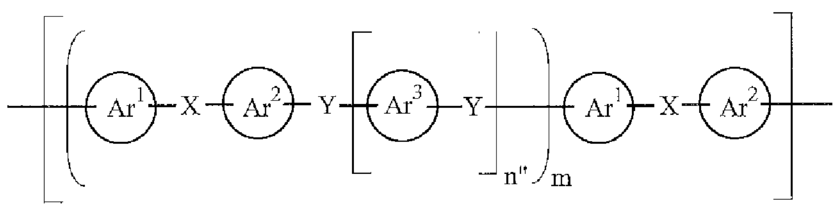
(L-1)

In Formula (L-1) above, m is a number of a repeating unit shown in round brackets included in the molecular chain (= divalent group represented by Formula (L-1) above), that is, the average degree of polymerization, and is, for example, from 2 to 50, preferably from 3 to 40, more preferably from 4 to 30, particularly preferably from 5 to 20, and most preferably from 5 to 10. In the case where m is less than the range, the strength and heat resistance of the resulting cured product tend to be insufficient. On the other hand, in the case where m is greater than the range, the glass transition temperature tends to be high. In addition, the solvent solubility tends to decrease. Furthermore, the melt viscosity of the curable compound product (that is, the viscosity when the curable compound product is melted) and the solution viscosity

of the curable compound product (that is, the viscosity of a solution obtained by dissolving the curable compound product in a solvent) tend to become too high, and molding tends to become difficult. Note that the value of m can be determined by GPC measurement or spectrum analysis of NMR. Furthermore, n" in Formula (L-1) above represents an integer of 0 or greater, and $Ar^1$ to $Ar^3$ are the same as those described above. A plurality of $Ar^1$ moieties in Formula (L-1) above represent the same groups. The same applies to $Ar^2$ and $Ar^3$.

L in Formula (1) is especially preferably a divalent group represented by Formula (L-1-1) or Formula (L-1-2) below. $Ar^3$ and Y in the following formula are the same as described above.

[Chem. 18]

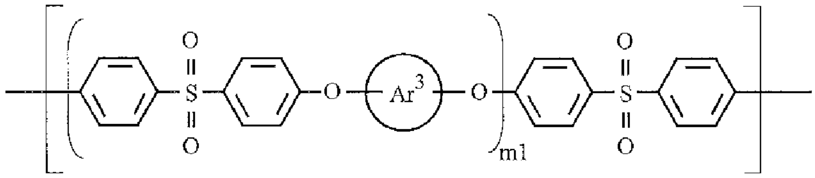

(L-1-1)

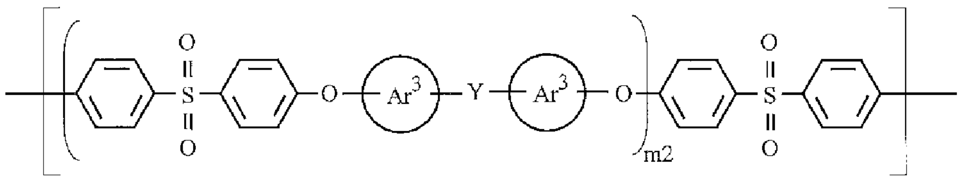

(L-1-2)

In the above formulas, m 1 and m2 indicate the number of repeating units shown in round brackets included in the molecular chain represented by Formula (L-1-1) above or Formula (L-1-2) below, that is, the average degree of polymerization, and are each, for example, from 2 to 50, preferably from 3 to 40, more preferably from 4 to 30, particularly preferably from 5 to 20, most preferably from 5 to 10, and especially preferably 5 or greater and less than 10. Note that the values of m1 and m2 can be determined by GPC measurement or spectrum analysis of NMR.

When m1 and m2 described above are less than the range described above, the resulting cured product tends to be brittle, and the mechanical properties tend to decrease. Furthermore, when m1 and m2 described above are greater than the range described above, workability tends to decrease due to, for example, a decrease in solubility in a solvent and an increase in melt viscosity.

The group represented by [-$Ar^3$-Y-$Ar^3$-] in Formula (L-1-2) above is preferably a group selected from the groups represented by the following Formulas (a-6) to (a-9). Note that, in the following formulas, positions of bonding are not particularly limited.

[Chem. 19]

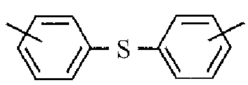

(a-6)

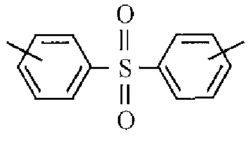

(a-7)

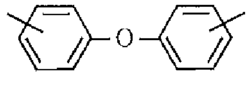

(a-8)

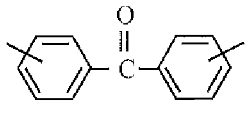

(a-9)

A proportion of the amount of the compound represented by Formula (1) above in the total amount of the curable compound product is, for example, 80 wt.% or greater, preferably 90 wt.% or greater, particularly preferably 95 wt.% or greater, and most preferably 98 wt.% or greater. Note that the upper limit is 100 wt.%.

In the curable compound product, the proportion (proportion expressed by the following equation, hereinafter, may be referred to as a "ring closure rate") of the group represented by Formula (r-1) above to the sum of the group represented by Formula (r-1) above and the group represented by Formula (r-2) above is 97% or greater, preferably 98% or greater, particularly preferably 98.5% or greater, and most preferably 99% or greater. When the ring closure rate of the curable compound product is within the above described range, the curable compound product exhibits excellent storage stability.

Ring closure rate= [(number of moles of group represented by Formula (r-1) above)/(number of moles of the group represented by Formula (r-1) above + number of moles of group represented by Formula (r-2) above)] × 100

The number of moles of each group can be determined by calculation from the peak area corresponding to each group in the $^1$H-NMR spectrum.

Furthermore, the number of moles of the group represented by Formula (r-1) (hereinafter, may be referred to as the "functional group concentration") per gram of the curable compound product is, for example, from $0.5 \times 10^{-4}$ to $20 \times 10^{-4}$ mol/g. The upper limit of the functional group concentration is preferably $15 \times 10^{-4}$ mol/g, most preferably $10 \times 10^{-4}$ mol/g. The lower limit of the functional group concentration is preferably $1.0 \times 10^{-4}$ mol/g, most preferably $1.5 \times 10^{-4}$ mol/g. When the functional group concentration of the curable compound product falls within the range described above, a cured product having excellent solvent solubility and excellent toughness and heat resistance can be formed. On the other hand, when the functional group concentration is less than the range described above, the solvent solubility tends to decrease. Further, when the functional group concentration is greater than the range described above, forming a cured product with excellent toughness tends to be difficult.

The functional group concentration is obtained by determining the area of each peak from the $^1$H-NMR spectrum of the curable compound product and substituting the determined value into the following equation:

Functional group concentration = [peak area of group represented by Formula (r-1)/number of protons in group represented by Formula (r-1)]/Σ[(each peak area/number of protons in group to which each peak is assigned) × chemical formula weight corresponding to each peak]

Furthermore, the number of moles (hereinafter, sometimes referred to as "unclosed ring concentration") of the group represented by Formula (r-2) above (that is, group including an amic acid structure) per gram of the curable compound product is, for example, $0.15 \times 10^{-4}$ mol/g or less, and preferably $0.10 \times 10^{-4}$ mol/g or less.

When the unclosed ring concentration of the curable compound product is within the above range, the curable compound product exhibits excellent storage stability. In addition, the exothermic onset temperature is high, and moldability is excellent. Further, since a ring-closing reaction curing reaction is suppressed during the curing reaction, a cured product exhibiting excellent toughness and heat resistance can be formed. On the other hand, when the unclosed ring concentration exceeds the range described above, the storage stability tends to decrease. In addition, a curing reaction attributed to the unclosed ring structure advances, and therefore the exothermic onset temperature tends to decrease. Furthermore, a ring-closing reaction may occur due to heating during molding, and water may be produced, and therefore molding defects such as the formation of voids in the molded article may occur.

The unclosed ring concentration is obtained by determining the area of each peak from the $^1$H-NMR spectrum of the curable compound product and substituting the determined value into the following equation:

Unclosed ring concentration = (peak area of group represented by Formula (r-2)/number of protons in group represented by Formula (r-2))/$\Sigma$[(each peak area/number of protons in group to which each peak is assigned) $\times$ chemical formula weight corresponding to each peak]

Furthermore, the curable compound product may contain an alkali metal derived from a raw material. When the alkali metal content (when two or more alkali metals are contained, the total content thereof) is reduced to, for example, 500 ppm by weight or less (preferably 100 ppm by weight or less, particularly preferably 50 ppm by weight or less, and most preferably 35 ppm by weight or less), a cured product having excellent heat resistance can be formed, and thus the alkali metal content is preferably reduced to such a range. Furthermore, when the alkali metal content exceeds the range described above, Tas tends to decrease. In other words, the heat resistance of the cured product that is obtained tends to decrease.

Method of Producing Curable Compound Product

The curable compound product can be produced by reacting a compound represented by Formula (2) below:

[Chem. 20]

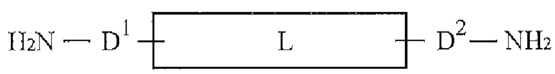 (2)

where in Formula (2), $D^1$ and $D^2$ are identical or different, and each represent a single bond or a linking group; L represents a divalent group having a repeating unit containing a structure represented by Formula (I) below and a structure represented by Formula (II) below:

[Chem. 21]

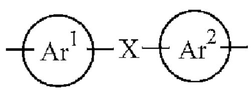 (I)

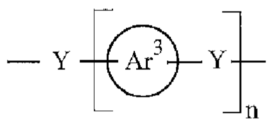 (II)

where in Formula (I) and Formula (II), $Ar^1$ to $Ar^3$ are identical or different, and each represent a group in which two hydrogen atoms are removed from a structure of an aromatic ring or a group in which two hydrogen atoms are removed from a structure containing two or more aromatic rings, the aromatic rings being bonded through a single bond or a linking group; X represents -CO-, -S-, or $-SO_2-$; Y is identical or different, and each represents -S-, $-SO_2-$, -O-, -CO-, -COO-, or -CONH-; and n represents an integer of 0 or greater; with the proviso that, of all X's and Y's included in L, at least one is $-SO_2-$, and a compound represented by the following Formula (3):

[Chem. 22]

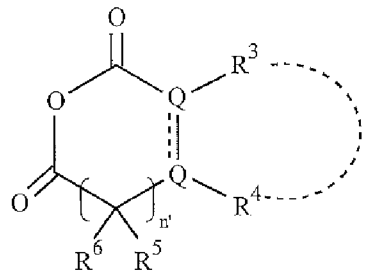

(3)

where in Formula (3), Q represents C or CH; two Q's in each formula bond to each other via a single bond or a double bond; $R^3$ to $R^6$ are identical or different, and each represent a hydrogen atom or a hydrocarbon group; $R^3$ and $R^4$ may bond to each other to form a ring; and n' represents an integer of 0 or greater.

When the compound represented by Formula (2) is reacted with the compound represented by Formula (3), the curable compound product is obtained via a two-step reaction as shown by the following scheme. Note that the $D^1$ substituent side of the compound represented by Formula (2) is shown in the following scheme, but the same reaction proceeds on the $D^2$ substituent side. $L^1$, $D^1$, Q, $R^3$ to $R^6$, and n' in the following scheme are the same as described above.

[Chem. 23]

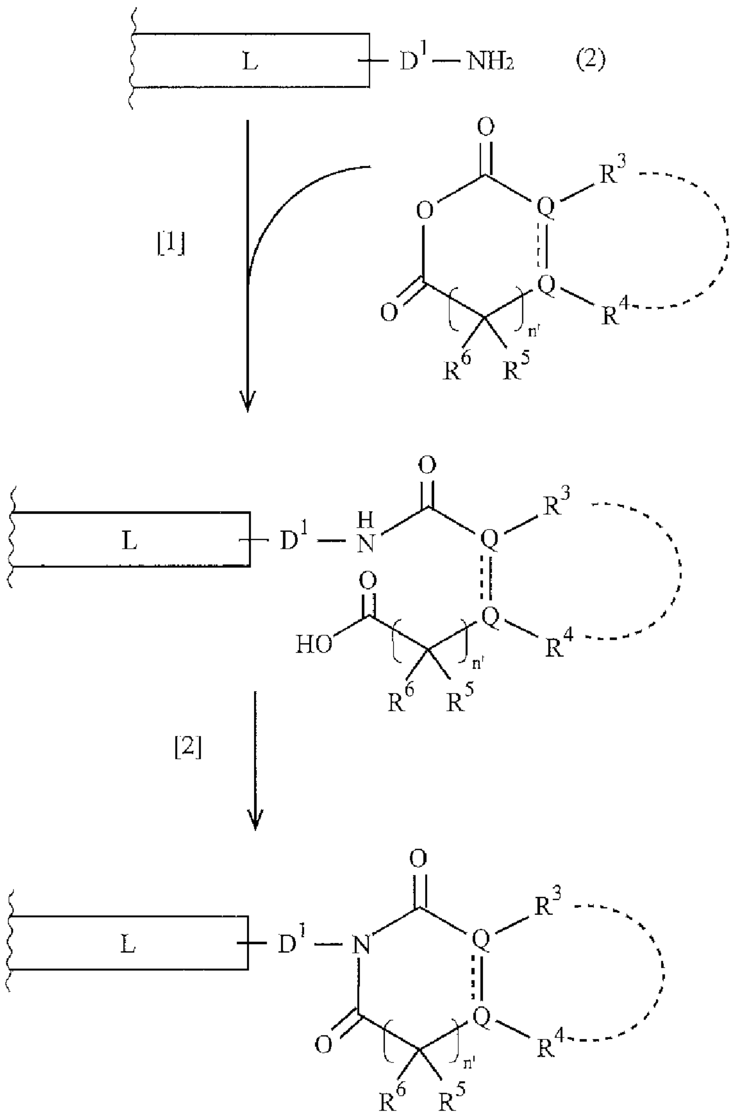


(2)

(3)

This reaction can be performed in the presence of a solvent. Examples of the solvent include N,N-dimethylformamide, N,N-dimethylacetamide, and N-methyl-2-pyrrolidone. One of these can be used alone or two or more in combination.

First Stage

The reaction at the first stage is a reaction in which the compound represented by Formula (3) reacts with an $NH_2$ group, which is a terminal group of the compound represented by Formula (2), and the terminal group is converted to a group represented by Formula (r-2). This reaction provides a compound represented by Formula (1) in which $R^1$ and $R^2$ are groups represented by Formula (r-2).

The used amount of the compound represented by Formula (3) is, for example, approximately from 2.0 to 4.0 mol per mol of the compound represented by Formula (2).

This reaction can be performed at room temperature (from 1 to 40°C). The reaction time is, for example, approximately from 1 to 30 hours. In addition, this reaction can be performed by any method, such as a batch method, a semi-batch method, and a continuous method.

Furthermore, as a raw material for the compound represented by Formula (2), the compound represented by Formula (3) or the like, a material having a low alkali metal content is preferably selected and used because a curable compound product having an alkali metal content not greater than 500 ppm by weight (preferably not greater than 100 ppm by weight, particularly preferably not greater than 50 ppm by weight, and most preferably not greater than 35 ppm by weight) can be obtained. The curable compound product having an alkali metal content reduced to the range described above can form a cured product having excellent heat resistance.

Second Stage; Ring Closing Reaction

The reaction at the second stage is a reaction in which the group represented by Formula (r-2) as the terminal group is converted to a group represented by Formula (r-1) through a ring closing reaction. This reaction provides a compound represented by Formula (1) in which at least either $R^1$ or $R^2$ is a group represented by Formula (r-1).

The reaction can proceed by heating at a temperature of 200°C or higher or by addition of a catalyst.

Among these, the addition of a catalyst is preferable in that the ring closing reaction can proceed while suppressing the progression of the curing reaction, and a curable compound product having a high ring closure rate and excellent storage stability is obtained. As the catalyst, a base catalyst, an acid catalyst, or the like can be used. Among these, it is desirable to advance the reaction using an acid catalyst as the catalyst in that side reactions are suppressed and a curable compound product in which contamination of a side reaction product is suppressed can be produced.

Examples of the base catalyst include amine compounds and sodium acetate. One of these can be used alone or two or more in combination.

Examples of the acid catalyst include inorganic acids such as hydrochloric acid, hydrogen bromide, hydrogen iodide, sulfuric acid, sulfuric anhydride, nitric acid, phosphoric acid, phosphorous acid, phosphorous tungstic acid, and phosphorous molybdic acid; sulfonic acids such as methanesulfonic acid, ethanesulfonic acid, trifluoromethanesulfonic acid, benzenesulfonic acid, and p-toluenesulfonic acid (pTSA); carboxylic acids such as acetic acid and oxalic acid; halogenated carboxylic acids such as chloroacetic acid, dichloroacetic acid, trichloroacetic acid, fluoroacetic acid, difluoroacetic acid, and trifluoroacetic acid; solid acids such as silica, alumina, and activated clay; and cationic ion exchange resins. One of these can be used alone or two or more in combination. Among these, at least one type selected from p-toluenesulfonic acid, methanesulfonic acid, sulfuric acid, and phosphoric acid is preferably used as the acid catalyst.

The usage amount of the base catalyst is, for example, from 0.01 to 1.0 mol, preferably from 0.02 to 0.5 mol, and particularly preferably from 0.05 to 0.4 mol, per mole of the compound represented by Formula (3).

The usage amount of the acid catalyst is, for example, from 0.01 to 1.0 mol, preferably from 0.02 to 0.5 mol, and particularly preferably from 0.05 to 0.4 mol, per mole of the compound represented by Formula (3).

The catalyst concentration in the reaction system is, for example, from 0.003 to 0.10 mmol/g, preferably from 0.005 to 0.07 mmol/g, and particularly preferably from 0.007 to 0.04 mmol/g. When the used amount of the catalyst falls below the range described above, the ring closure rate of the curable compound product tends to decrease, and the storage stability in solution tends to decrease as the ring closure rate decreases.

In addition, in the reaction described above, quick removal of byproduct water generated by the reaction to the outside of the reaction system is preferable in order to further promote the progress of the ring closing reaction. Examples of the method of removing byproduct water include a method of using a dehydrating agent such as a carboxylic acid anhydride and a method of using a solvent that azeotropes with water. In these cases, the byproduct water removal efficiency may vary depending on the shape and space volume of the reaction vessel, the piping layout, and the thermal insulation state.

However, when carboxylic anhydride is used as a dehydrating agent, a side reaction proceeds simultaneously with the advancement of the ring closing reaction, and a large amount of side reaction products are produced. For example, when a reaction between the compound represented by Formula (2) and maleic anhydride is carried out while dehydration is performed using acetic anhydride, side reaction products represented by Formulas (I-a) and (I-b) are produced together with a compound represented by Formula (I), which is the target compound. Furthermore, when such side reaction products are mixed into the curable compound product, the exothermic onset temperature tends to decrease. Therefore, when dehydration is performed using a carboxylic acid anhydride, it is preferable to suppress the progress of the side reaction by adjusting the used amount of raw material, the method of supplying the raw material, and the supply rate thereof, and to sufficiently purify the product.

When an acid catalyst is used, the water produced by the ring closing reaction is preferably removed by an azeotropic reaction. Examples of azeotropic agents that can be used include benzene, toluene, xylene, and ethylbenzene.

It is preferable that the ratio (former/latter; weight ratio) of the used amount of the azeotropic agent to the used amount of the solvent is, for example, in the range from 25/75 to 45/55, because it is possible to promote the progress of the reaction, and the effect of improving the ring closure rate of the curable compound product can be obtained.

After completion of this reaction, the resulting reaction product can be separated and purified by typical precipitation, washing, and filtration.

Preparation of Compound Represented by Formula (2)

Of the compounds represented by Formula (2) above used as the raw materials for the curable compound product, a compound represented by Formula (2-1) below, for example, can be produced via the following steps [1-1] and [1-2].

Step [1-1]: A compound represented by Formula (4) below and a compound represented by Formula (5) below

are allowed to react in the presence of a base to form a compound represented by Formula (6) below.

Step [1-2]: An aminoalcohol (a compound represented by Formula (7) below) is allowed to react with the compound represented by Formula (6) below:

[Chem. 24]

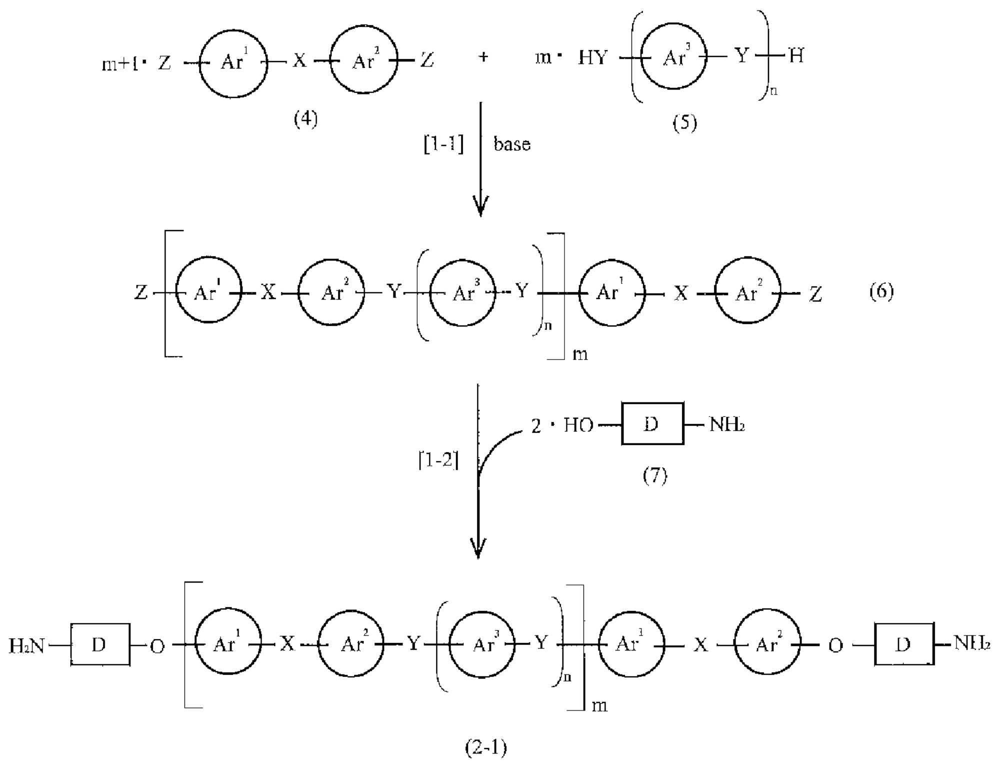


In the above formulas, $Ar^1$ to $Ar^3$, X, Y, and n are the same as those described above. D represents a linking group, and examples of the linking group include the same groups that are exemplified for the linking group in $D^1$ and $D^2$. m is the average degree of polymerization of the repeating unit and is, for example, from 3 to 50, preferably from 4 to 30, and particularly preferably from 5 to 20. Z represents a halogen atom.

Step [1-1]

Examples of the compound represented by Formula (4) above include halides of diphenyl sulfone and derivatives thereof.

Examples of the compound represented by Formula (5) above include hydroquinone, resorcinol, catechol, 2,6-naphthalenediol, 2,7-naphthalenediol, 1,5-naphthalenediol, 4,4'-dihydroxybiphenyl, 4,4'-dihydroxydiphenyl ether, 4,4'-dihydroxybenzophenone, 4,4'-dihydroxydiphenyl sulfide, 4,4'-dihydroxydiphenyl sulfone, bisphenol A, bisphenol F, bisphenol S, 2,5-dihydroxybiphenyl, and derivatives thereof. One of these can be used alone or two or more in combination.

Examples of the derivatives include compounds in which a substituent is bonded to an aromatic hydrocarbon group of the compound represented by Formula (4) above or the compound represented by Formula (5). Examples of the substituent include alkyl groups having from 1 to 6 carbons, alkoxy groups having from 1 to 6 carbons, and halogen atoms.

The used amount of the compound represented by Formula (4) is desirably adjusted in a range of 1 mole or greater, based on the average degree of polymerization of the molecular chain in the desired compound represented by Formula (6), in a range of 1 mole or greater, per mol of the compound represented by Formula (5). For example, per mol of the compound represented by Formula (5), approximately 1.2 mol (e.g., from 1.19 to 1.21 mol) of the compound represented by Formula (4) is preferably used in the case of the average degree of polymerization of 5, approximately 1.1 mol (e.g., from 1.09 to 1.11 mol) of the compound represented by Formula (4) is preferably used in the case of the average degree of polymerization of 10, and approximately 1.05 mol (e.g., from 1.04 to 1.06 mol) of the compound represented by Formula (4) is preferably used in the case of the average degree of polymerization of 20.

The reaction of step [1-1] is performed in the presence of a base (e.g., at least one selected from the group consisting of inorganic bases, such as sodium hydroxide, potassium hydroxide, calcium hydroxide, sodium carbonate, potassium carbonate, and sodium hydrogencarbonate, and organic bases, such as pyridine and triethylamine). The used amount of the base can be appropriately adjusted based on the type of the base. For example, the used amount of diacidic base, such as calcium hydroxide, is approximately from 1.0 to 2.0 mol per mol of the compound represented by Formula (5).

Furthermore, the reaction of step [1-1] can be performed in the presence of a solvent. As the solvent, for example, an organic solvent, such as N-methyl-2-pyrrolidone, dimethylformamide or dimethyl sulfoxide, or a solvent mixture of two or more thereof can be used.

The used amount of the solvent is, for example, approximately from 5 to 20 times in weight relative to the total amount (weight) of the compound represented by Formula (4) and the compound represented by Formula (5). When the solvent is used in an amount greater than the range described above, the reaction rate tends to decrease.

The reaction atmosphere in step [1-1] is not particularly limited as long as it does not inhibit the reaction. For example, a nitrogen atmosphere, an argon atmosphere, or any other atmosphere may be used.

The reaction temperature in step [1-1] is, for example, approximately from 100 to 200°C. The reaction time is, for example, approximately from 3 to 24 hours. In addition, this reaction can be performed by any method, such as a batch method, a semi-batch method, and a continuous method.

After completion of this reaction, the resulting reaction product can be separated and purified by typical precipitation, washing, and filtration.

Step [1-2]

Examples of the compound represented by Formula (7) above include 4-aminophenol, 2-amino-6-hydroxynaphthalene, and regioisomers and derivatives thereof. One of these can be used individually, or two or more in combination.

The used amount of the compound represented by Formula (7) above can be appropriately adjusted based on the average degree of polymerization of the molecular chain in the desired compound represented by Formula (2-1). For example, the amount may be adjusted to a range approximately from 0.4 to 0.6 mol per mol of the compound represented by Formula (5) in the case of the average degree of polymerization of 5, approximately from 0.2 to 0.4 mol per mol of the compound represented by Formula (5) in the case of the average degree of polymerization of 10, and approximately from 0.1 to 0.15 mol per mol of the compound represented by Formula (5) in the case of the average degree of polymerization of 20.

The reaction in step [1-2] is preferably carried out in the presence of a base. Examples of the base include inorganic bases, such as sodium hydroxide, potassium hydroxide, calcium hydroxide, sodium carbonate, potassium carbonate, and sodium hydrogencarbonate, and organic bases, such as pyridine and triethylamine. One of these can be used alone or two or more in combination.

The used amount of the base can be appropriately adjusted based on the type of the base. For example, the used amount of monoacidic base, such as sodium hydroxide, is approximately from 1.0 to 3.0 mol per mol of the compound represented by Formula (7) above.

Furthermore, the reaction in step [1-2] can be performed in the presence of a solvent. As the solvent, the same solvent as that used in step [1] can be used.

The reaction atmosphere in step [1-2] is not particularly limited as long as it does not inhibit the reaction. For example, a nitrogen atmosphere, an argon atmosphere, or any other atmosphere may be used.

The reaction temperature in step [1-2] is, for example, approximately from 100 to 200°C. The reaction time is, for example, approximately from 1 to 15 hours. In addition, this reaction can be performed by any method, such as a batch method, a semi-batch method, and a continuous method.

After completion of this reaction, the resulting reaction product can be separated and purified by typical precipitation, washing, and filtration.

Furthermore, the compound represented by Formula (2-1) above can also be produced by collectively charging and reacting a compound represented by Formula (4) above, a compound represented by Formula (5) above, and a compound represented by Formula (7) above.

The curable compound product can also be produced by performing a dehydrative imidization reaction between a compound represented by Formula (7) above and a compound represented by Formula (3) above to yield a compound (8) in which an amino group ($-NH_2$) of the compound represented by Formula (7) above has been converted to Formula (r-1) above, and then collectively charging and reacting the compound (8) together with a compound represented by Formula (4) above and a compound represented by Formula (5). However, in this method, even if the compound (8) does not include Formula (r-2) above, the group represented by Formula (r-1) above is opened by the water produced during the reaction to form a group represented by Formula (r-2) above. Therefore, also when the production is performed

by this method, it is preferable to perform the reaction while removing the water produced in the reaction system, for example, by azeotropic dehydration, and to stop the reaction after confirming that the reaction has progressed sufficiently, for example, by a method such as sampling to confirm the ring closure rate.

Curable Composition

The curable composition according to an embodiment of the present disclosure contains one type or two or more types of the curable compound products described above. The content of the curable compound product (in the case where two or more types are contained, the total amount thereof) in the total amount of the curable composition (or the total amount of non-volatile contents of the curable composition) is, for example, 30 wt.% or greater, preferably 50 wt.% or greater, particularly preferably 70 wt.% or greater, and most preferably 90 wt.% or greater. Note that the upper limit is 100 wt.%. That is, the curable composition according to an embodiment of the present disclosure may be formed from the curable compound product alone.

The curable composition may contain another component as necessary in addition to the curable compound product. Examples of such another component include curable compound products other than the curable compound product described above, catalysts, fillers, organic resins (such as silicone resins, epoxy resins, and fluororesins), solvents, stabilizers (such as antioxidants, ultraviolet absorbers, light-resistant stabilizers, and heat stabilizers), flame retardants (such as phosphorus-based flame retardants, halogen-based flame retardants, and inorganic flame retardants), flame retardant aids, reinforcing materials, nucleating agents, coupling agents, lubricants, waxes, plasticizers, release agents, impact resistance modifiers, hue modifiers, fluidity improvers, colorants (such as dyes and pigments), dispersants, antifoaming agents, defoaming agents, antimicrobial agents, preservatives, viscosity modifiers, and thickeners. Of these, a single type may be used alone, or two or more types thereof may be used in combination.

The curable composition may contain a curable compound other than the curable compound product described above; however, the proportion of the curable compound product in the total amount (100 wt.%) of curable compounds contained in the curable composition is, for example, 70 wt.% or greater, preferably 80 wt.% or greater, and particularly preferably 90 wt.% or greater. Note that the upper limit is 100 wt.%.

Since the curable compound product has excellent solvent solubility, the curable composition may be a solvent-dissolved material, in which the curable compound product is dissolved in a solvent. As the solvent, a solvent for which the curable compound product exhibits good solubility (for example, at least one type of solvent selected from ethers, chain ketones, cyclic ketones, amides, halogenated hydrocarbons, and sulfoxides) is preferred, and at least one type of solvent selected from chain ketones, cyclic ketones, amides, and halogenated hydrocarbons is especially preferred.

Furthermore, even when the curable composition does not contain any crosslinking agent or curing accelerator (even when the total content of the crosslinking agent and the curing accelerator in the total amount of the curable composition is, for example, 3 wt.% or less, and preferably less than 1 wt.%), a cured product can be rapidly formed. Therefore, the resulting cured product has ultra-high heat resistance. Furthermore, because the content of an unreacted curing accelerator and decomposition products in the cured product can be suppressed to an extremely low level, outgassing originated from these can be suppressed.

Also, because the curable composition contains the curable compound product, the curable composition rapidly cures when subjected to a heating treatment, and can form a cured product having ultra-high heat resistance. Note that the heating treatment conditions can be appropriately set in the same range as that for the curing conditions of the curable compound product described above.

The curable composition described above can be suitably used as molding materials for composite materials (such as fiber-reinforced plastics and prepregs) to be used in a severe environmental temperature, such as those for electronic information devices, home appliances, automobiles, precision machines, aircraft, devices for the space industry, and energy field (oil drill pipes/tubes and fuel containers), and as functional materials, such as shielding materials, conducting materials (such as thermally conducting materials), insulating materials, and adhesives (such as heat-resistant adhesives). In addition, the curable composition can be suitably used as sealing agents, paints, inks, sealants, resists, shaping materials, and forming materials [forming materials for, for example, automobile components, such as thrust washers, oil filters, seals, bearings, gears, cylinder head covers, bearing retainers, intake manifolds, and pedals; components of semiconductor and liquid crystal producing apparatuses, such as base materials, electrical insulation materials (such as electrical insulation films), laminated plates, electronic papers, touch panels, solar cell substrates, optical waveguide materials, light guide plates, holographic memories, silicon wafer carriers, IC chip trays, electrolytic capacitor trays, and insulating films; optical components, such as lenses; compressor components, such as pumps, valves, and seals; cabin interior components of aircraft; medical device components and components of food and beverage producing facilities, such as sterilized devices, columns, and piping; and members for electric and electronic devices as represented by housings to be used for personal computers and cell phones, and keyboard supporters being members to support keyboards inside personal computers].

Because the curable composition has the characteristics described above, especially, the curable composition

can be suitably used as a sealing agent that covers a semiconductor element in a highly heat-resistant and highly voltage-resistant semiconductor device (such as power semiconductor), for which employment of a known resin material has been difficult.

Furthermore, because the curable composition has the characteristics described above, the curable composition can be suitably used as an adhesive [e.g., heat-resistant adhesive used for laminating a semiconductor in a highly heat-resistant and highly voltage-resistant semiconductor device (such as power semiconductor)].

Furthermore, because the curable composition has the characteristics described above, the curable composition can be suitably used as a paint (solvent-type coating agent or powder coating agent) [e.g., paint (solvent-type coating agent or powder coating agent) used for a highly heat-resistant and highly voltage-resistant semiconductor device (such as power semiconductor)].

Molded Product

The molded product according to an embodiment of the present disclosure includes a cured product or semi-cured product of the curable compound product described above. The shape of the molded product is not particularly limited, and may be appropriately selected depending on the application, and the molded product may be planar or three-dimensional. Furthermore, it may be in a pellet form or in a particulate form. The molded product can be produced by subjecting the curable compound product (or the curable composition) to a molding method, such as injection molding, transfer molding, compression molding, or extrusion molding.

The molded product has excellent heat resistance. Therefore, the composite material can be suitably used as a material for replacing a metal, such as iron and aluminum, in the fields of housing and building, sporting goods, automobiles, and aircraft and aerospace industry. In particular, a molded product in a planer form (or a molded product in a film form) can be suitably used as an interlayer insulating film of an electric device.

Laminate

The laminate according to an embodiment of the present disclosure has a structure in which a cured product or semi-cured product of the curable compound product and a substrate are laminated. The laminate includes structures that are a cured product or semi-cured product of the curable compound product/substrate and a substrate/cured product or semi-cured product of the curable compound product/sub strate.

Examples of the materials of the substrate include semiconductor materials (such as ceramics, SiC, and gallium nitride), paper, coated paper, plastic films, wood, fabric, nonwoven fabric, and metals (such as stainless steel, aluminum alloy, and copper).

The laminate has a configuration in which the substrates are laminated via an adhesive layer containing a cured product or semi-cured product of the curable compound product and having excellent heat resistance and adhesion to the substrate. The laminate can be suitably used as, for example, an electric circuit board.

The laminate can be produced, for example, by placing the curable compound product on a substrate and performing a heating treatment.

The laminate can also be produced by applying a molten material of the curable compound product onto a support made of plastic, solidifying the applied molten material to form a thin film containing the curable compound product, detaching the formed thin film from the support, laminating the formed thin film on a substrate, and subjecting to a heating treatment.

Composite Material

The composite material according to an embodiment of the present disclosure includes a cured product or semi-cured product of the curable compound product and a fiber. The shape of the composite material is not particularly limited, and examples thereof include a fiber form and a sheet form.

Examples of the fiber include carbon fibers, aramid fibers, and glass fibers. One of these can be used alone or two or more in combination. The fiber may be processed into a thread form or a sheet form (woven fabric or nonwoven fabric).

The composite material can be produced by, for example, impregnating fibers with a solution prepared by dissolving the curable compound product in a solvent or impregnating fibers with a molten material of the curable compound product and performing a heating treatment. The composite material formed by semi-curing the impregnated curable compound product through the heating treatment can be suitably used as an intermediate product, such as a prepreg.

The composite material has a configuration, in which the curable compound product is incorporated into gaps between fibers and cured therein, and has a light weight and high strength as well as excellent heat resistance. Therefore,

the composite material can be suitably used as a material for replacing a metal, such as iron and aluminum, in the fields of housing and building, sporting goods, automobiles, and aircraft and aerospace industry. In addition, the composite material can be suitably used as clothing material for fire fighting (fire fighting clothing, clothing for activity, clothing for rescue and heat resistant clothing); curtains and footcloth; separators, such as separators for secondary batteries and separators for fuel cells; filters, such as industrial filters, filters for cars, and medical filters; and space materials.

Each of the configurations, their combinations, and the like of the present disclosure above is an example, and addition, omission, substitution, and change of the configuration can be appropriately made without departing from the gist of the present disclosure. In addition, the present disclosure is not limited by the embodiments and is limited only by the claims.

Examples

Hereinafter, the present disclosure will be described more specifically with reference to examples, but the present disclosure is not limited by these examples.

Note that the measurements were performed under the following conditions.

NMR Measurement

Measuring instrument: JEOL ECA 500 or BRUKER AVANCE 600 MHz
Measurement solvent: deuterated DMSO, deuterated chloroform or a liquid mixture of deuterated chloroform/pentafluorophenol (PFP) = 2/1 (wt/wt)
Chemical shift: TMS as the reference

GPC Measurement

Apparatus: pump "LC-20AD" (available from Shimadzu Corporation)
Detector: RID-10A (available from Shimadzu Corporation) or MODEL 302 TDA (available from Viscotek Corporation) and UV 2501 (available from Viscotek Corporation)
Solvent: THF or chloroform
Column: Shodex KF-803 + Shodex KF802 + Shodex KF801 × 2
Flow rate: 1.0 mL/min
Temperature: 40°C
Sample concentration: 0.1% (wt/vol)
calibrated with polystyrene standard

DSC Measurement

Apparatus: TA Instruments Q2000
Rate of temperature increase: 20°C/min
Atmosphere: nitrogen atmosphere

TGA Measurement

Apparatus: Seiko Instruments TG/DTA 6200
Rate of temperature increase: 20°C/min
Atmosphere: nitrogen atmosphere

Example 1 (Production of Compound Product (1-1))

Step 1-1

A reactor provided with a stirrer, a nitrogen introducing tube, and a Dean-Stark apparatus was charged with 70.00 g of 4,4'-dichlorodiphenyl sulfone (DCDPS), 46.38 g of bisphenol A (BisA), 42.11 g of anhydrous potassium carbonate ($K_2CO_3$), 463.2 g of N-methylpyrrolidone (NMP), and 284.2 g of toluene (Tol), the mixture was heated while stirring in a nitrogen atmosphere, and the toluene was refluxed at 130 to 140°C for 4 hours. Subsequently, the contents were further heated to distill off toluene at 170 to 180°C. Furthermore, stirring was continued at 170 to 180°C for 10 hours, after which the temperature was returned to room temperature.

Step 1-2

Subsequently, 9.310 g of 4-aminophenol (4-AP), 11.790 g of anhydrous potassium carbonate ($K_2CO_3$), 38.6 g of N-methylpyrrolidone (NMP), and 343.7 g of toluene (Tol) were added to the reactor containing the reaction product, after which the mixture was heated again while stirring in a nitrogen atmosphere, and the toluene was refluxed at 130 to 140°C for 3 hours. Subsequently, heating was performed to distill off the toluene at 170 to 180°C, and stirring was further continued for 4 hours while the above temperature was maintained. The mixture was then cooled to room temperature, and the reaction solution was added to 3000 mL of methanol and filtered, and thereby a powdery solid was obtained. This powdery solid was repeatedly washed with methanol and water, and then dried overnight at 80°C under reduced pressure, and thereby a powdery solid of diamine-1 (compound represented by the following formula with m in the formula being 5) was obtained.

[Chem. 25]

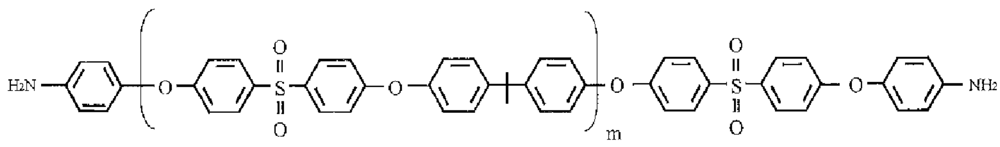

Diamine-1

Step 2

A reactor provided with a stirrer, a nitrogen introducing tube, and a Dean-Stark apparatus was charged with 100.00 g of the diamine-1 obtained in step 1 as a compound (2), 11.12 g of maleic anhydride (MAH), 285.6 g of N-methylpyrrolidone (NMP), and 141.6 g of toluene (Tol), and the mixture was stirred in a nitrogen atmosphere at room temperature for 5 hours. Subsequently, 1.798 g of p-toluenesulfonic acid (pTSA) was added as a catalyst, and the temperature was increased to 140°C, after which stirring was continued for 8 hours, the toluene was refluxed, and moisture was removed. The reaction solution was brought back to room temperature, and then added to 3000 mL of methanol, and thus a powdery solid was formed. This powdery solid was repeatedly washed with methanol and water and then dried overnight at 80°C under reduced pressure, and thereby 98.8 g of a compound product (1-1) (including a compound represented by the following Formula (1-1)) was obtained. The properties of the obtained compound product are shown in Table 2 below.

[Chem. 26]

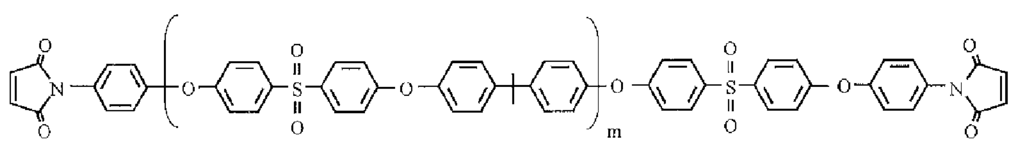

(1-1)

Example 2 (Production of Compound Product (1-2))

A compound product (1-2) (including a compound represented by the following Formula (1-2)) was obtained in the same manner as in Example 1 with the exception that the conditions were changed as described in the following Table 1. The properties of the obtained compound product are shown in Table 2 below.

[Chem. 27]

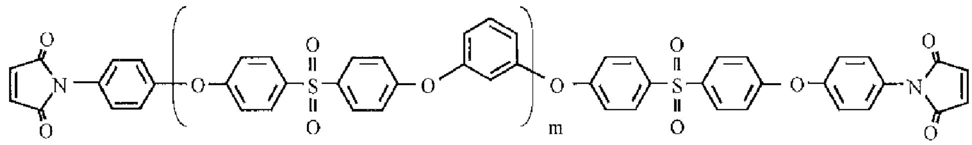

(1-2)

Example 3 (Production of Compound Product (1-3))

A compound product (1-3) (including a compound represented by the following Formula (1-3)) was obtained in the same manner as in Example 1 with the exception that the conditions were changed as described in the following Table 1. The properties of the obtained compound product are shown in Table 2 below.

[Chem. 28]

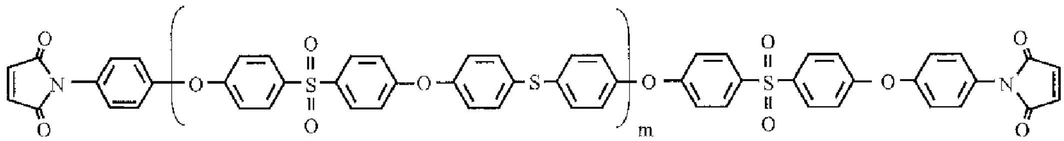

(1-3)

Example 4 (Production of Compound Product (1-4))

A compound product (1-4) (including a compound represented by the following Formula (1-4)) was obtained in the same manner as in Example 1 with the exception that the conditions were changed as described in the following Table 1. The properties of the obtained compound product are shown in Table 2 below.

[Chem. 29]

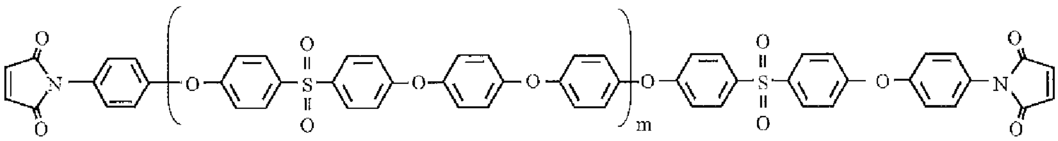

(1-4)

Example 5 (Production of Compound Product (1-5))

A compound product (1-5) (including a compound represented by the following Formula (1-5)) was obtained in the same manner as in Example 1 with the exception that the conditions were changed as described in the following Table 1. The properties of the obtained compound product are shown in Table 2 below.

[Chem. 30]

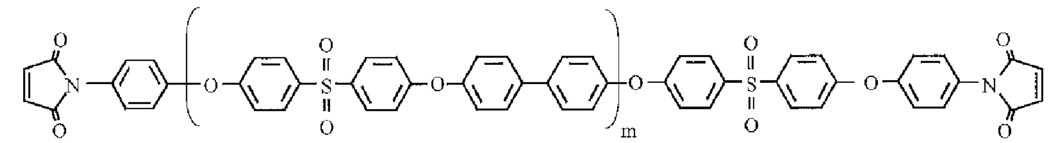

(1-5)

Comparative Example 1 (Production of Compound Product (1-6))

A compound product (1-6) (including a compound represented by the Formula (1-5)) was obtained in the same manner as in Example 1 with the exception that the conditions were changed as described in the following Table 1. The properties of the obtained compound product are shown in Table 2 below.

Comparative Example 2 (Production of Compound Product (1-7))

A compound product (1-7) (including a compound represented by the Formula (1-4)) was obtained in the same manner as in Example 1 with the exception that the conditions were changed as described in the following Table 1. The properties of the obtained compound product are shown in Table 2 below.

Comparative Example 3 (Production of Compound Product (1-8))

The same procedures as in Example 1 were carried out with the exception that the conditions were changed as described in Table 1. Although turbidity of the reaction solution was observed in step 2, the procedures were carried out in that state, and a compound product (1-8) (including a compound represented by the following formula (1-8)) was obtained. The properties of the obtained compound product are shown in Table 2 below.

[Chem. 31]

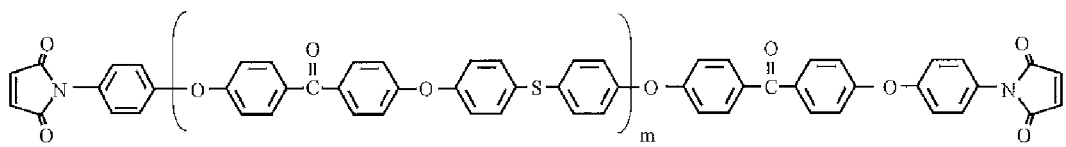

(1-8)

Comparative Example 4 (Production of Compound Product (1-9))

The same procedures as in Example 1 were carried out with the exception that the conditions were changed as described in Table 1. Although turbidity of the reaction solution was observed in step 2, the procedures were carried out in that state, and a compound product (1-9) (including a compound represented by the following formula (1-9)) was obtained. The properties of the obtained compound product are shown in Table 2 below.

[Chem. 32]

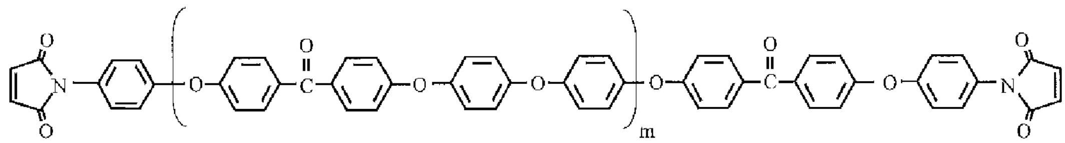

(1-9)

Comparative Example 5 (Production of Compound Product (1-10))

The same procedures as in Example 1 were carried out with the exception that the conditions were changed as described in Table 1. Although turbidity was observed in the reaction solution in steps 1-1 and 1-2, the steps were completed in that state. The compound obtained in the above steps was not dissolved in NMP, and therefore step 2 could not be carried out. Therefore, a compound product (1-10) (including a compound represented by the following formula (1-10)) was not obtained.

[Chem. 33]

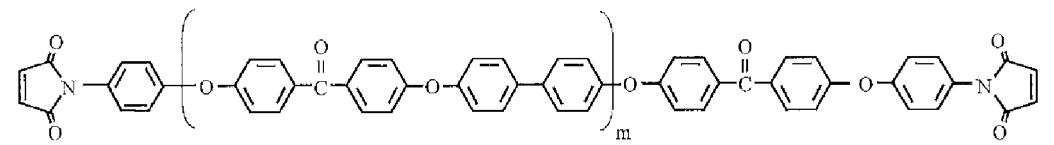

(1-10)

Comparative Example 6 (Production of Compound Product (1-11))

A compound product (1-11) (including a compound represented by the following Formula (1-11)) was obtained in the same manner as in Example 1 with the exception that the conditions were changed as described in the following Table 1. The properties of the obtained compound product are shown in Table 2 below.

[Chem. 34]

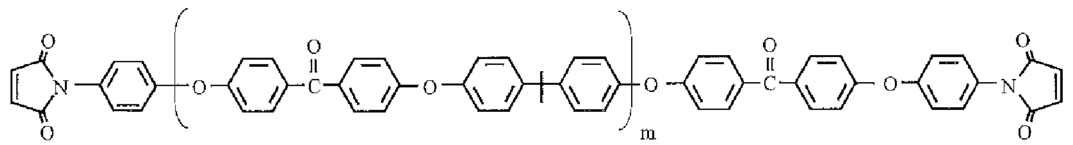

(1-11)

Comparative Example 7 (Production of Compound Product (1-12))

A compound product (1-12) (including a compound represented by the following Formula (1-12)) was obtained in the same manner as in Example 1 with the exception that the conditions were changed as described in the following Table 1. The properties of the obtained compound product are shown in Table 2 below.

[Chem. 35]

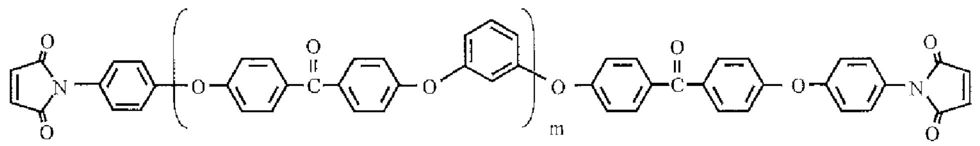

(1-12)

Comparative Example 8 (Production of Compound Product (1-13))

As described in Table 1 below, steps 1-1 and 1-2 were carried out under the same conditions as in Example

4, and a powdered solid of diamine-2 was obtained.

In step 2, a reactor provided with a stirrer and a nitrogen introducing tube was charged with 100.00 g of diamine-2 obtained in the abovementioned steps, 11.70 g of maleic anhydride (MAH), and 288.5 g of N-methylpyrrolidone (NMP), and the mixture was stirred in a nitrogen atmosphere at room temperature for 5 hours. Subsequently, 20.30 g of acetic anhydride and 0.330 g of sodium acetate were added, and the mixture was stirred at 60°C for 6 hours. The reaction solution was brought back to room temperature, and then added to 3000 mL of methanol, and thereby a powdery solid was formed. This powdery solid was repeatedly washed with methanol and water and then dried overnight at 80°C under reduced pressure, and thereby 97.2 g of a compound product (1-13) (including a compound represented by Formula (1-4)) was obtained. The properties of the obtained compound product are shown in Table 2 below.

Table 1

Table 1

| | Compound product | Step 1-1 | | | | | | | Step 1-2 | | | | Step 2 | | | | | |
|---|---|---|---|---|---|---|---|---|---|---|---|---|---|---|---|---|---|---|
| | | Compound (4) | | Compound (5) | | $K_2CO_3$ | NMP | Tol | 4-AP | $K_2CO_3$ | NMP | Tol | Compound (2) | MAH | NMP | Tol | Catalyst | |
| | | Type | g | Type | g | g | g | g | g | g | g | g | g | g | g | g | Type | g |
| Example 1 | 1-1 | DCDPS | 70.00 | BisA | 46.38 | 42.11 | 463.2 | 284.2 | 9.310 | 11.790 | 38.6 | 343.7 | 100.00 | 11.12 | 285.6 | 141.6 | pTSA | 1.798 |
| Example 2 | 1-2 | DCDPS | 93.54 | RS | 29.89 | 56.27 | 503.9 | 308.9 | 12.443 | 15.711 | 67.2 | 391.8 | 92.44 | 13.24 | 308.3 | 130.3 | pTSA | 1.530 |
| Example 3 | 1-3 | DCDPS | 86.15 | DHDPS | 54.57 | 51.82 | 556.8 | 341.3 | 11.459 | 14.523 | 74.2 | 432.9 | 110.28 | 12.50 | 367.9 | 155.5 | pTSA | 1.850 |
| Example 4 | 1-4 | DCDPS | 70.00 | DHDPE | 41.08 | 42.11 | 442.1 | 271.2 | 9.311 | 11.792 | 38.5 | 329.2 | 100.00 | 11.70 | 288.5 | 148.9 | pTSA | 1.891 |
| Example 5 | 1-5 | DCDPS | 73.50 | DHBP | 39.72 | 44.22 | 472.1 | 289.6 | 9.777 | 12.382 | 42.4 | 352.4 | 100.00 | 12.08 | 290.4 | 153.8 | pTSA | 1.953 |
| Comparative Example 1 | 1-6 | DCDPS | 59.68 | DHBP | 35.18 | 39.16 | 387.0 | 237.4 | 4.330 | 5.488 | 32.2 | 287.1 | 35.51 | 2.35 | 308.1 | 130.1 | pTSA | 0.310 |
| Comparative Example 2 | 1-7 | DCDPS | 70.00 | DHDPE | 41.08 | 42.11 | 442.1 | 271.2 | 9.311 | 11.792 | 38.5 | 329.2 | 100.00 | 11.70 | 288.5 | 119.1 | pTSA | 0.378 |
| Comparative Example 3 | 1-8 | DFBP | 49.10 | DHDPS | 40.93 | 38.86 | 372.1 | 228.3 | 8.594 | 10.892 | 29.7 | 254.0 | 75.82 | 9.38 | 456.9 | 203.0 | pTSA | 1.850 |
| Comparative Example 4 | 1-9 | DFBP | 49.09 | DHDPE | 37.91 | 38.86 | 341.1 | 209.2 | 8.594 | 10.892 | 32.4 | 277.1 | 73.25 | 9.38 | 588.6 | 261.5 | pTSA | 1.920 |
| Comparative Example 5 | 1-10 | DFBP | 34.37 | DHBP | 24.44 | 27.20 | 260.5 | 159.8 | 6.016 | 7.624 | 18.9 | 161.6 | 49.49 | 6.56 | 397.7 | 176.7 | pTSA | 1.850 |
| Comparative Example 6 | 1-11 | DFBP | 55.00 | BisA | 47.96 | 43.54 | 451.9 | 190.7 | 5.040 | 6.390 | 61.6 | 260.1 | 48.57 | 5.88 | 390.3 | 173.4 | pTSA | 0.761 |
| Comparative Example 7 | 1-12 | DFBP | 59.27 | RS | 24.91 | 46.91 | 323.0 | 198.0 | 10.380 | 13.200 | 43.1 | 251.4 | 70.00 | 11.21 | 233.5 | 99.0 | pTSA | 1.450 |
| Comparative Example 8 | 1-13 | DCDPS | 70.00 | DHDPE | 41.08 | 42.11 | 442.1 | 271.2 | 9.311 | 11.792 | 38.5 | 329.2 | 100.00 | 11.70 | 288.5 | 0.0 | NaOAc | 0.330 |

Abbreviations in Table 1 are described below:

<Compound (4)>

DCDPS: 4,4'-dichlorodiphenyl sulfone
DFBP: 4,4'-difluorobenzophenone

<Compound (5)>

BisA: bisphenol A
RS: resorcinol
DHDPS: 4,4'-dihydroxydiphenyl sulfide
DHDPE: 4,4'-dihydroxydiphenyl ether
DHBP: 4,4'-dihydroxybiphenyl

For the compound products obtained in the Examples and the Comparative Examples, the functional group concentration, the unclosed ring concentration, and the ring closure rate were calculated from the integrated intensity ratio of the signal in the $^1$H-NMR spectrum. Furthermore, the number average molecular weight and weight average molecular weight were determined by GPC measurement. Furthermore, Tg and calorific value were determined by DSC measurement, and the 5% thermal weight loss temperature (Tas) was determined by TGA measurement.

The $^1$H-NMR measurement results of the compound product (1-5) obtained in Example 5 and the compound product (1-6) obtained in Comparative Example 1 are illustrated in FIG. 1. When an amic acid with an unclosed ring was included, a signal appeared in a range from 6.2 to 6. 5 Ppm. However, it is clear that the intensity of the signal derived from the amic acid is significantly lower in the compound product (1-5) in comparison to the compound product (1-6).

The TGA measurement results of the compound products (1-4) and (1-5) obtained in the Examples are illustrated in FIG. 2. From FIG. 2, it is clear that each of the compound products obtained in the Examples have a $T_{d5}$ of 400°C or higher and excel in heat resistance.

The storage stability of each of the obtained compound products was evaluated by the following method.

Each of the compound products was dried under reduced pressure at 80°C overnight, then brought back to room temperature in dry nitrogen, and used as a sample. The residual moisture content (measured by the Karl Fischer method) in the compound products after drying was 0.05% or less in all cases.

In addition, a predetermined amount of the sample was weighed into a vial in which the inside was replaced with dry nitrogen, and NMP (moisture-reduced product) was added to form a 20 wt.% NMP solution.

The resulting 20 wt.% NMP solution was stored in a desiccator (temperature: 23°C) for 10 days, and the storage stability was evaluated from the ratio of the pre-storage viscosity ($\eta_0$) to the post-storage viscosity ($\eta_{10}$). The viscosity was measured with an E-type viscometer (Brookfield Viscometer LVDT3T) while maintaining the measurement temperature at 23°C using a constant temperature bath (Huber MPC-K6).

The concentration of alkali metals contained in the compound product was measured by ICP emission spectrometry (using the Agilent 5110 available from Agilent Technologies, Inc.).

As a sample, 1 g of the compound product was diluted 100-fold with NMP to prepare a test solution for ICP-AES measurements.

A commercially available K1000 standard solution for atomic absorption spectrometry and a commercially available Na1000 standard solution were appropriately diluted with NMP and used as standard solutions for calibration curves.

The results are summarized and shown in Table 2 below.

Table 2

Table 2

| | Compound product | Molecular weight | | Functional group concentration (μmol/g) | Ring closure rate (%) | Unclosed ring concentration (μmol/g) | Tg (°C) | Exothermic Onset Temperature (°C) | Calorific value (J/g) | $T_{d5}$ (°C) | Alkali metal (ppm) | | Pre-storage viscosity ($\eta_0$) | Pre-storage/poststorage viscosity ratio ($\eta_{10}/\eta_0$) |
|---|---|---|---|---|---|---|---|---|---|---|---|---|---|---|
| | | Mn | Mw | | | | | | | | Na | K | mPa•s | |
| Example 1 | 1-1 | 2990 | 5050 | 444.4 | 99.7 | 1.4 | 162 | 270 | 39 | 480 | 13 | 11 | 22.3 | 1.1 |
| Example 2 | 1-2 | 2500 | 3500 | 688.0 | 100.0 | 0.0 | 140 | 292 | 58 | 490 | <10 | <10 | 14.4 | 1.0 |
| Example 3 | 1-3 | 2950 | 5050 | 523.6 | 99.6 | 2.2 | 145 | 287 | 56 | 476 | <10 | <10 | 18.4 | 1.2 |
| Example 4 | 1-4 | 3500 | 6000 | 503.1 | 100.0 | 0.0 | 153 | 294 | 52 | 503 | <10 | <10 | 28.8 | 1.1 |
| Example 5 | 1-5 | 3280 | 6330 | 502.7 | 100.0 | 0.0 | 174 | 251 | 51 | 499 | <10 | 12 | 38.4 | 1.0 |
| Comparative Example 1 | 1-6 | 3800 | 10050 | 228.9 | 86.2 | 36.6 | 189 | 234 | 13 | 488 | 15 | <10 | 72.0 | 6.7 |
| Comparative Example 2 | 1-7 | 3610 | 6150 | 486.2 | 96.0 | 20.8 | 154 | 242 | 60 | 482 | <10 | <10 | 32.2 | 3.8 |
| Comparative Example 3 | 1-8 | *1 | *1 | *1 | *1 | *1 | *2 | *2 | *2 | 479 | *3 | *3 | *3 | *3 |
| Comparative Example 4 | 1-9 | *1 | *1 | *1 | *1 | *1 | *2 | *2 | *2 | 432 | *3 | *3 | *3 | *3 |
| Comparative Example 5 | 1-10 | - | - | - | - | - | - | - | - | - | - | - | - | - |
| Comparative Example 6 | 1-11 | 4160 | 8510 | 503.5 | 96.2 | 19.8 | 124 | 240 | 36 | 505 | <10 | <10 | 29.7 | 3.2 |
| Comparative Example 7 | 1-12 | 2650 | 4100 | 876.4 | 99.5 | 4.4 | 115 | 268 | 61 | 499 | <10 | <10 | 16.0 | 1.3 |
| Comparative Example 8 | 1-13 | 3450 | 6200 | 496.6 | 99.5 | 2.3 | 151 | 197 | 50 | 396 | 350 | 95 | 33.8 | 1.1 |

* 1: Partially insoluble in chloroform, and thus measurement was not possible.
*2: An endothermic peak was observed and overlapped with Tg and an exothermic peak, and thus an evaluation could not be implemented.
*3: Partially insoluble in NMP at room temperature, and thus measurement was not possible.

Solvent Solubility Evaluation

A compound product (0.5 g) obtained in an Example or Comparative Example was mixed with a solvent (50 g) indicated in the following table, and stirred for 24 hours, and the solubility in the solvent was evaluated based on the following criteria.

Evaluation Criteria

Good: Completely dissolved by stirring at room temperature (23°C) for 24 hours
Marginal: Completely dissolved by stirring for 24 hours while heating at a temperature of 50°C or higher
Poor: At least a portion remained undissolved

The results are summarized and shown in Table 3 below.

[Table 3]

Table 3

| | Compound product | Solubility | | |
|---|---|---|---|---|
| | | NMP | DMAc | $CHCl_3$ |
| Example 1 | 1-1 | Good | Good | Good |
| Example 2 | 1-2 | Good | Good | Good |
| Example 3 | 1-3 | Good | Good | Good |
| Example 4 | 1-4 | Good | Good | Good |
| Example 5 | 1-5 | Good | Good | Good |
| Comparative Example 1 | 1-6 | Good | Good | Good |
| Comparative Example 2 | 1-7 | Good | Good | Good |
| Comparative Example 3 | 1-8 | Marginal | Poor | Poor |
| Comparative Example 4 | 1-9 | Marginal | Poor | Poor |
| Comparative Example 5 | 1-10 | - | - | - |
| Comparative Example 6 | 1-11 | Good | Good | Good |
| Comparative Example 7 | 1-12 | Good | Good | Good |
| Comparative Example 8 | 1-13 | Good | Good | Good |

Solvent

NMP: N-methyl-2-pyrrolidone
DMAc: dimethylacetamide
$CHCl_3$: chloroform

Melt Press Molding

A mold was filled with a respective compound product obtained in the Examples and the Comparative Examples, and then placed in a press machine (30-ton manual hydraulic vacuum press, IMC-46E2-3 type, available from Imoto Machinery Co., Ltd.). The temperature was increased from 50°C to 280°C at 20°C/min in a vacuum and maintained at

280°C for 1 hour, after which the temperature was further increased to 320°C at 20°C/min and maintained for 30 minutes. Subsequently, the press machine was cooled, the mold was removed when the temperature of the press machine reached 100°C or lower, and a flat plate-shaped molded body (thickness: 1.0±0.1 mm) formed from a cured product of the respective compound product was obtained. The Tg of the obtained molded body was measured.

The results are summarized and shown in Table 4 below.

[Table 4]

Table 4

| | Compound product | Press-molded body Tg (°C) |
|---|---|---|
| Example 1 | 1-1 | 186 |
| Example 2 | 1-2 | 185 |
| Example 3 | 1-3 | 181 |
| Example 4 | 1-4 | 194 |
| Example 5 | 1-5 | 229 |
| Comparative Example 1 | 1-6 | 220 |
| Comparative Example 2 | 1-7 | 191 |
| Comparative Example 3 | 1-8 | 214 |
| Comparative Example 4 | 1-9 | 150 |
| Comparative Example 5 | 1-10 | - |
| Comparative Example 6 | 1-11 | 173 |
| Comparative Example 7 | 1-12 | 152 |
| Comparative Example 8 | 1-13 | 189 |

As a summary of the above, configurations and variations of the present disclosure are described below.

[1] A curable compound having the following characteristics (a) to (g):

(a) a number average molecular weight calibrated with a polystyrene standard is from 1000 to 15000;
(b) a proportion of a structure derived from an aromatic ring in a total amount of the curable compound is 50 wt.% or greater;
(c) solvent solubility at 23°C is 1 g/100 g or greater;
(d) a glass transition temperature is from 80 to 230°C;
(e) a viscosity ($\eta_0$) of a 20 wt.% NMP solution obtained by subjecting the curable compound product to a reduced-pressure drying process and then dissolving the reduced-pressure-dried curable compound product in NMP, and a viscosity ($\eta_{10}$) of the 20 wt.% NMP solution after being left to stand for 10 days in a desiccator maintained at 23°C satisfy Equation (E) below:

$$\eta_{10}/\eta_0 < 2 \quad (E)$$

(f) a molecular skeleton has a sulfonyl group; and
(g) an exothermic onset temperature is 220°C or higher.

[2] The curable compound product according to [1], further including the following characteristic (h):
(h) a 5% weight loss temperature ($T_{d5}$) measured at a temperature increase rate of 20°C/min in a nitrogen atmosphere is 400°C or higher.
[3] The curable compound product according to [1] or [2], further including the following characteristic (i):

(i) a nitrogen atom content is from 2.8 to 0.1 wt.%.

[4] The curable compound product according to any one of [1] to [3], further including the following characteristic (j): (j) a 5% weight loss temperature ($T_{d5}$) measured at a temperature increase rate of 20°C/min in a nitrogen atmosphere is 400°C or higher.
[5] The curable compound product according to any one of [1] to [4], further including the following characteristic (k): (k) an elastic modulus measured by a method in accordance with JIS K7161 is 1000 Mpa or higher.
[6] The curable compound product according to any one of [1] to [5], further including the following characteristic (l): (l) a yield point stress measured by a method in accordance with JIS K7161 is 70 MPa or higher.
[7] The curable compound product according to any one of [1] to [6], further including the following characteristic (m): (m) an elongation at break measured by a method in accordance with JIS K7161 is 10% or more.
[8] A curable compound product wherein a proportion of a group represented by Formula (r-1) to a sum of the group represented by Formula (r-1) and a group represented by Formula (r-2) is 97% or greater.
[9] The curable compound product according to [8], in which $D^1$ and $D^2$ in Formula (1) are identical or different, and each represent a group selected from groups having structures represented by Formulas (d-1) to (d-4).
[10] The curable compound product according to [8] or [9], in which the group represented by Formula (r-1) is a group represented by Formula (r-1').
[11] The curable compound product according to [8] or [9], in which the group represented by Formula (r-1) is a group selected from groups represented by Formulas (r-1-1) to (r-1-6).
[12] The curable compound product according to [8] or [9], in which the group represented by Formula (r-1) is a group selected from groups represented by Formula (r-1-1) or (r-1-5).
[13] The curable compound product according to any one of [8] to [12], in which the group represented by Formula (r-2) is a group represented by Formula (r-2').
[14] The curable compound product according to any one of [8] to [12], in which the group represented by Formula (r-2) is a group obtained by subjecting an imide bond portion in a group represented by Formulas (r-1-1) to (r-1-6) to ring-opening.
[15] The curable compound product according to any one of [8] to [12], in which the group represented by Formula (r-2) is a group obtained by subjecting an imide bond portion in a group represented by Formula (r-1-1) or Formula (r-1-5) to ring-opening.
[16] The curable compound product according to any one of [8] to [15], in which the $R^1$-$D^1$- group and the $R^2$-$D^2$- group in Formula (1) are identical or different, and are each a group represented by Formulas (rd-1'-1), (rd-1'-2), (rd-2'-1) or (rd-2'-2).
[17] The curable compound product according to any one of [8] to [16], in which L in Formula (1) is a divalent group represented by Formula (L-1).
[18] The curable compound product according to any one of [8] to [16], in which L in Formula (1) is a divalent group represented by Formula (L-1-1) or (L-1-2).
[19] The curable compound product according to any one of [8] to [18], in which $Ar^1$ to $Ar^3$ in Formula (I) and Formula (II) are identical or different, and each represent a group in which two hydrogen atoms are removed from a structure of an aromatic ring having from 6 to 14 carbons, or a group in which two hydrogen atoms are removed from a structure containing two or more aromatic rings each having from 6 to 14 carbons, the aromatic rings being bonded through a single bond, a linear or branched-chain alkylene group having from 1 to 5 carbons, or a group in which one or more hydrogen atoms of a linear or branched-chain alkylene group having from 1 to 5 carbons are substituted with a halogen atom.
[20] The curable compound product according to any one of [1] to [19], in which the number of moles for the group represented by Formula (r-1) per gram of the curable compound product is from $0.5 \times 10^{-4}$ to $20 \times 10^{-4}$ mol/g.
[21] The curable compound product according to any one of [1] to [20], in which the number of moles for the group represented by Formula (r-2) per gram of the curable compound product is $0.15 \times 10^{-4}$ mol/g or less.
[22] The curable compound product according to any one of [1] to [21], having an alkali metal content of 500 ppm by weight or less.
[23] A molded product including a cured product of the curable compound product described in any one of [1] to [22].
[24] A molded product including a semi-cured product of the curable compound product described in any one of [1] to [22].
[25] A molded product including a cured product or semi-cured product of the curable compound product described in any one of [1] to [22].
[26] A laminate including a configuration in which a cured product or semi-cured product of the curable compound product described in any one of [1] to [22] and a substrate are laminated.
[27] A method of producing a laminate, the method including placing the curable compound product described in any one of [1] to [22] on a substrate and subjecting to a heating treatment to form a laminate having a configuration

in which a cured product or semi-cured product of the curable compound product and the substrate are laminated.
[28] The method of producing a laminate according to [27], the method including applying a molten material of the curable compound product onto a support made of plastic, solidifying the applied material to obtain a thin film containing the curable compound product, detaching the formed thin film from the support, laminating the formed thin film on a substrate, and subjecting to a heating treatment.
[29] A composite material including a cured product or a semi-cured product of the curable compound product described in any one of [1] to [22] and fibers.
[30] A method for producing a composite material, the method including using the curable compound product described in any one of [1] to [22], and producing a composite material containing fibers and a cured product or a semi-cured product of the curable composition.
[31] Use of the curable compound product described in any one of [1] to [22] to produce a composite material containing fibers and a cured product or a semi-cured product of the curable composition.
[32] An adhesive including the curable compound product described in any one of [1] to [22].
[33] A paint including the curable compound product described in any one of [1] to [22].
[34] A sealing agent including the curable compound product described in any one of [1] to [22].
[35] Use of the curable compound product described in any one of [1] to [22], as an adhesive.
[36] Use of the curable compound product described in any one of [1] to [22], as a paint.
[37] Use of the curable compound product described in any one of [1] to [22], as a sealing agent.

Industrial Applicability

The curable compound product according to an embodiment of the present disclosure has excellent solvent solubility. Furthermore, a solution obtained by dissolving the curable compound product in a solvent has high storage stability. In addition, since the curable compound product has a low melt viscosity and a high exothermic onset temperature, when the curable compound product is subjected to melting and molding, the degree of freedom in setting conditions relating to molding is high, curing during injection into a mold or the like can be prevented, and moldability is excellent. Further, a cured product of the curable compound product exhibits ultra-high heat resistance and excellent toughness.

Therefore, the curable compound product is suitable for use as a raw material in an adhesive, a sealing agent, a paint, or the like, which are used in electronic information devices, home appliances, automobiles, precision machines, aircraft, devices for the space industry, and the like.

## Claims

**1.** A curable compound product having the following characteristics (a) to (g):

(a) a number average molecular weight (calibrated with polystyrene standard) is from 1000 to 15000;
(b) a proportion of a structure derived from an aromatic ring in a total amount of the curable compound product is 50 wt.% or greater;
(c) solvent solubility at 23°C is 1 g/100 g or greater;
(d) a glass transition temperature is from 80 to 230°C;
(e) a viscosity ($\eta_0$) of a 20 wt.% NMP solution obtained by subjecting the curable compound product to a reduced-pressure drying process and then dissolving the reduced-pressure-dried curable compound product in NMP, and a viscosity ($\eta_{10}$) of the 20 wt.% NMP solution after being left to stand for 10 days in a desiccator maintained at 23°C satisfy Equation (E) below:

$$\eta_{10}/\eta_0 < 2 \quad (E)$$

(f) a molecular skeleton has a sulfonyl group; and
(g) an exothermic onset temperature is 220°C or higher.

**2.** A curable compound product comprising a compound represented by Formula (1) below:

[Chem. 1]

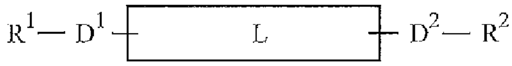

(1)

where in Formula (1), $R^1$ and $R^2$ are identical or different, and each represent a group represented by Formula (r-1) below or a group represented by Formula (r-2) below:

[Chem. 2]

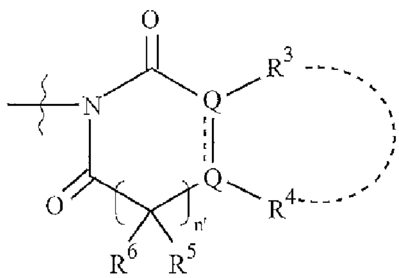

(r-1)

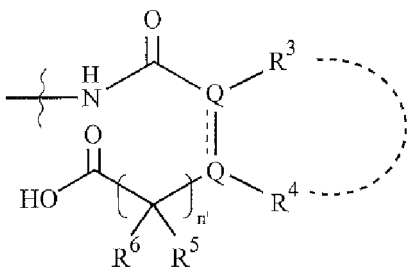

(r-2)

where in Formula (r-1) and Formula (r-2), Q represents C or CH, and in each formula, two Q's bond to each other via a single bond or a double bond; $R^3$ to $R^6$ are identical or different, and each represent a hydrogen atom or a hydrocarbon group; $R^3$ and $R^4$ may bond to each other to form a ring; n' represents an integer of 0 or greater; and a bond indicated by a wavy line in each formula is bonded to $D^1$ or $D^2$,
and in Formula (1), $D^1$ and $D^2$ are identical or different, and each represent a single bond or a linking group; L represents a divalent group having a repeating unit containing a structure represented by Formula (I) below and a structure represented by Formula (II) below:

[Chem. 3]

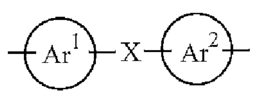

(I)

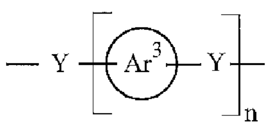

(II)

where in Formula (I) and Formula (II), $Ar^1$ to $Ar^3$ are identical or different, and each represent a group in which two hydrogen atoms are removed from a structure of an aromatic ring, or a group in which two hydrogen atoms are removed from a structure containing two or more aromatic rings bonded through a single bond or a linking group; X represents -CO-, -S-, or $-SO_2-$; Y is identical or different, and each represents -S-, $-SO_2-$, -O-, -CO-, -COO-, or - CONH-; and n represents an integer of 0 or greater,
with the proviso that, of all X's and Y's included in L, at least one is $-SO_2-$,

wherein a proportion of the group represented by Formula (r-1) above to a sum of the group represented by Formula (r-1) above and the group represented by Formula (r-2) above is 97% or greater.

**3.** The curable compound product according to claim 2, wherein $D^1$ and $D^2$ in Formula (1) are identical or different, and each represent a group selected from the group having structures represented by Formulas (d-1) to (d-4) below:

[Chem. 4]

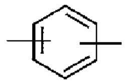

(d-1)

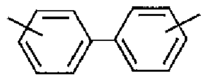

(d-2)

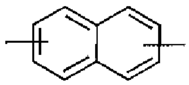

(d-3)

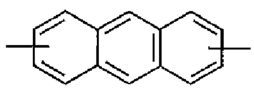

(d-4)

**4.** The curable compound product according to claim 2 or 3, wherein $Ar^1$ to $Ar^3$ in Formula (I) and Formula (II) are identical or different, and each represent a group in which two hydrogen atoms are removed from a structure of an aromatic ring having from 6 to 14 carbons, or a group in which two hydrogen atoms are removed from a structure containing two or more aromatic rings each having from 6 to 14 carbons, the aromatic rings being bonded through a single bond, a linear or branched-chain alkylene group having from 1 to 5 carbons, or a group in which one or more hydrogen atoms of a linear or branched-chain alkylene group having from 1 to 5 carbons are substituted with a halogen atom.

**5.** A molded product comprising a cured product or semi-cured product of the curable compound product described in any one of claims 1 to 4.

**6.** A laminate having a configuration in which a cured product or semi-cured product of the curable compound product described in any one of claims 1 to 4 and a substrate are laminated.

**7.** A method of producing a laminate, the method comprising placing the curable compound product described in any one of claims 1 to 4 on a substrate and subjecting to a heating treatment to form a laminate having a configuration in which a cured product or semi-cured product of the curable compound product and the substrate are laminated.

**8.** The method of producing a laminate according to claim 7, the method comprising applying a molten material of the curable compound product onto a support made of plastic, solidifying the applied material to obtain a thin film containing the curable compound product, detaching the formed thin film from the support, laminating the formed thin film on a substrate, and subjecting to a heating treatment.

**9.** A composite material comprising a cured product or semi-cured product of the curable compound product described in any one of claims 1 to 4 and fibers.

**10.** An adhesive comprising the curable compound product described in any one of claims 1 to 4.

**11.** A paint comprising the curable compound product described in any one of claims 1 to 4.

**12.** A sealing agent comprising the curable compound product described in any one of claims 1 to 4.

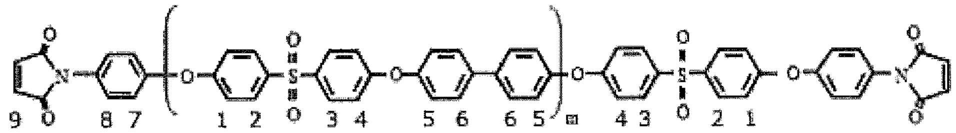

COMPOUND PRODUCT (1-5)

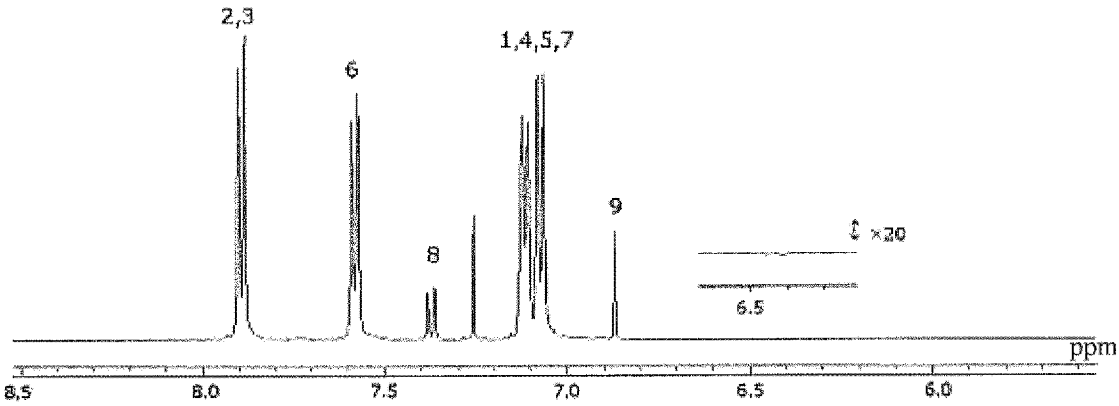


COMPOUND PRODUCT (1-6)

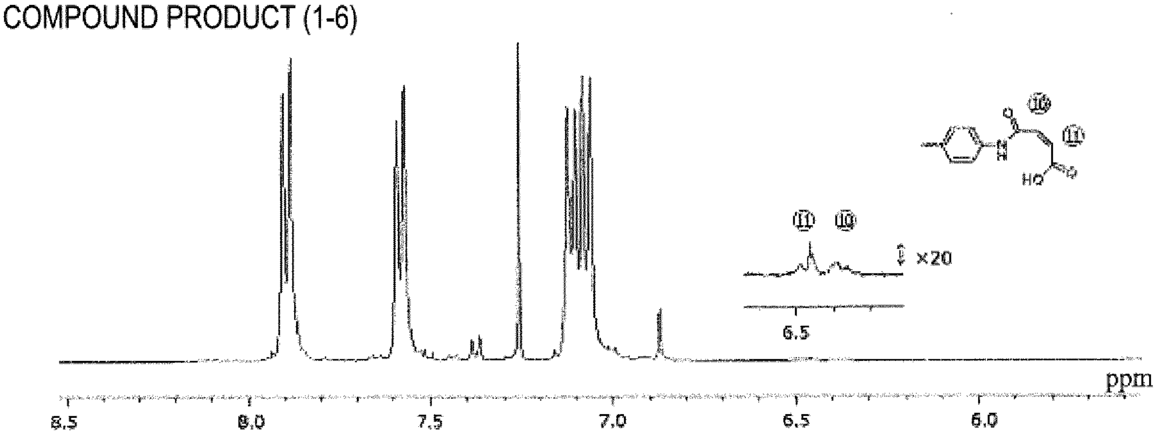


FIG. 1

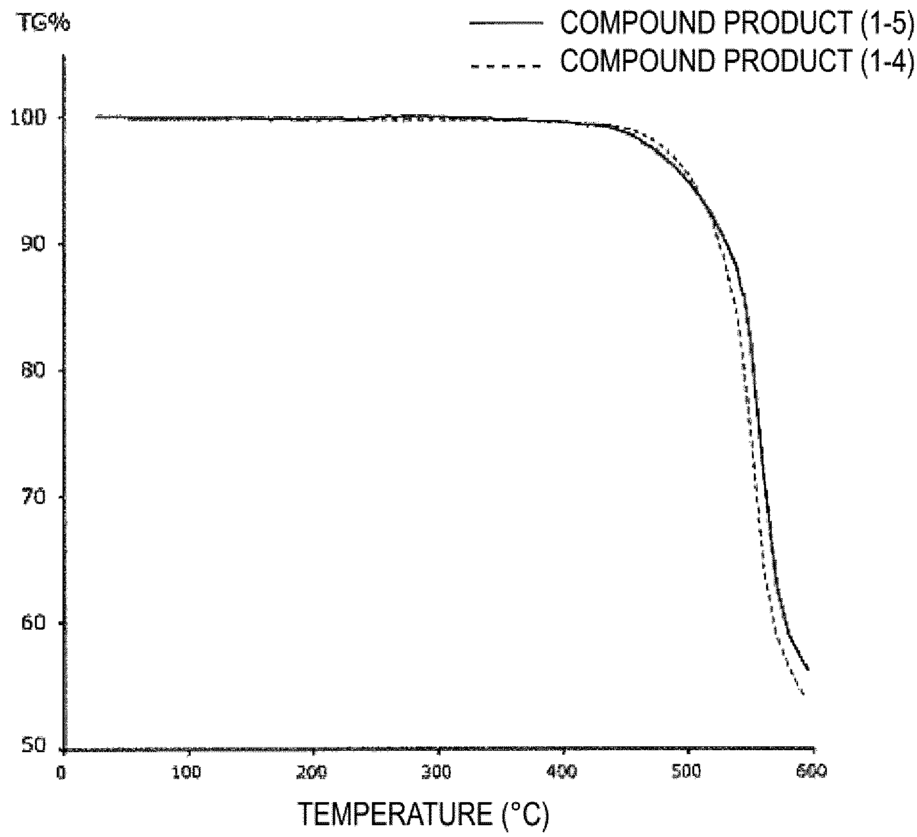
TG%
COMPOUND PRODUCT (1-5)
COMPOUND PRODUCT (1-4)
100
90
80
70
60
50
0
100
200
300
400
500
600
TEMPERATURE (°C)

FIG. 2

## INTERNATIONAL SEARCH REPORT

International application No.
**PCT/JP2021/036833**

**A. CLASSIFICATION OF SUBJECT MATTER**

***C07D 207/452***(2006.01)i; ***C08F 38/00***(2006.01)i; ***C08G 65/333***(2006.01)i; ***C08G 65/48***(2006.01)i; ***C09D 201/00***(2006.01)i; ***C09J 201/00***(2006.01)i; ***B32B 7/027***(2019.01)i
FI: C07D207/452; B32B7/027; C09J201/00; C09D201/00; C08G65/48; C08F38/00; C08G65/333

According to International Patent Classification (IPC) or to both national classification and IPC

**B. FIELDS SEARCHED**

Minimum documentation searched (classification system followed by classification symbols)

C07D207/452; C08F38/00; C08G65/333; C08G65/48; C09D201/00; C09J201/00; B32B7/027

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| Y | JP 2020-502310 A (DAICEL CORP) 23 January 2020 (2020-01-23) examples, paragraphs [0059], [0060], [0080]-[0091], fig. 21, tables 4, 5 | 1-12 |
| Y | JP 60-204758 A (TORAY INDUSTRIES) 16 October 1985 (1985-10-16) examples, page 1, right column, line 3 to page 2, upper right column, line 15 | 1-12 |

☐ Further documents are listed in the continuation of Box C.  ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **30 November 2021** | **14 December 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2021/036833**

| Patent document cited in search report | Publication date (day/month/year) | Patent family member(s) | Publication date (day/month/year) |
|---|---|---|---|
| JP 2020-502310 A | 23 January 2020 | US 2020/0079726 A1<br>examples, paragraphs [0115], [0116], [0137]-[0149], fig. 21, tables 4, 5<br>WO 2018/107453 A1<br>EP 3556793 A1<br>TW 201823289 A<br>KR 10-2019-0092537 A<br>CN 110268003 A | |
| JP 60-204758 A | 16 October 1985 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2020170858 A
- JP 2000219741 A
- JP 48036163 A
- JP 62151414 A
- WO 2019244694 A